# EUROPEAN PATENT SPECIFICATION

(11) **EP 3 326 651 B1**
(45) Date of publication and mention of the grant of the patent: **13.01.2021**
(21) Application number: 17202780.7
(22) Date of filing: 21.11.2017
(51) Int. Cl.: A61K 47/10, A61K 45/06, A61K 47/26, A61K 9/08, A61K 31/135, A61K 31/167

(54) **ACETAMINOPHEN AND TRAMADOL COMPOUND ORAL SOLUTION**
ORALE LÖSUNG AUS ACETAMINOPHEN UND TRAMADOLVERBINDUNG
SOLUTION ORALE DE COMPOSÉ D'ACÉTAMINOPHÈNE ET DE TRAMADOL

(30) Priority: 23.11.2016 TW 105138341
(43) Date of publication of application: 30.05.2018
(73) Proprietor: Bora Pharmaceuticals Co., Ltd., Taipei City (TW)
(72) Inventor: CHEN, Shih-Min, Taipei City (TW); LAI, Chun-Hsun, Taipei City (TW); LIN, Ying-Ku, Taipei City (TW); SHENG, Pao-Shi, Taipei City (TW)
(74) Representative: Becker & Kurig Partnerschaft Patentanwälte PartmbB

(56) References cited:
- EP-A2- 2 377 514
- WO-A1-2010/040652
- DE-A1-102006 056 458
- US-A1- 2014 275 151

## Description

The present invention relates to an acetaminophen and tramadol compound oral solution, especially to an acetaminophen and tramadol compound oral solution in a co-solvent system.

Pain treatment is a medical issue of great concern. Conventional compound analgesics include the compound tablet of acetaminophen (paracetamol, or P-hydroxyacetanilide) and tramadol, the interaction between compound ingredients of which provides superior effect over common compound prescriptions with less significant side-effects.

The convention tablet formulation, however, constitute difficulties in swallowing processes that denies access thereto of aged patients, dementia patients, cephalic cervical surgery patients, radiotherapy or chemotherapy patients, gastrointestinal patients, and oral esophageal patients, due to inconveniences or incapability.

The scope of clinical application of the conventional acetaminophen and tramadol compound tablet is limited by the need of chewing or powderizing processes preceding administration. Thus, the inconvenient or incapable patients who have difficulties in chewing or receiving externally fed powderized drug, may not avail themselves of the supreme analgesic effect of the conventional acetaminophen and tramadol compound tables.

Although an oral solution formulation may grant access of the above-described patients to the drugs, an oral solution formulation of acetaminophen and tramadol is not available in prior art. Acetaminophen dissolves poorly in water. Tramadol makes great aqueous solutions while hardly dissolves in acetone. Acetaminophen and tramadol barely co-exist in an oral solution as co-solutes.

In addition, a single treatment requires a dosage of acetaminophen of at least 325 milligram (mg), and at 25 degrees Celsius every milliliter (mL) of water solves no more than 14.00 mg of acetaminophen, which further requires that an acetaminophen and tramadol compound oral solution forcibly made with conventional technical skills forms a volume of as large as 25 to 30 mL per treatment. An oral solution of that large treatment volume, with the accompanying drinking water, unreasonably commands a patient to receive or be fed with 100 mL of liquid in a single treatment.

The foregoing formulation of large volume brings along significant difficulties for the aged, severe or dysphagia patients to bear, and becomes the source of the misery that makes acetaminophen and tramadol compound oral solution inapplicable.

As aforementioned, the manufacture and provision of conveniently applicable acetaminophen and tramadol compound oral solution, in the technical field, are a market need.

Additionally, tramadol is known for having a bitter taste, where acetaminophen is much more famously so. As that the above problem be dealt is desired, it is as desired that the bitter tastes be favorably flavored.

To overcome the shortcomings, the present invention provides an acetaminophen and tramadol compound oral solution to mitigate or obviate the aforementioned problems. The acetaminophen and tramadol compound oral solution in accordance with the present invention, taking a total weight of the acetaminophen and tramadol compound oral solution as 100 wt%, comprises:
an acetaminophen having a weight percentage of 1.447 wt% to 6.702 wt%;
a tramadol having a weight percentage of 0.167 wt% to 0.773 wt%; and
a solvent system, weighted 90.828 wt% to 98.02 wt%, comprising:
   a glycerin, a water and a polyethylene glycol (PEG), wherein the PEG has a weight percentage of 17 wt% to 65 wt% of the total weight of the acetaminophen and tramadol compound oral solution.

Manufacturing process of the conventional tablet requires 24 to 32 hours. In contrast, the oral solution formulation needs no more than 8 to 16 hours to produce. Therefore, in the aspects of manufacturing, quality control and cost management, the acetaminophen and tramadol compound oral solution in accordance with the present invention is also superior to the conventional analgesic tablets.

Besides, the acetaminophen and tramadol compound oral solution in accordance with the present may be polyer-packed or flavored. Even though the acetaminophen and tramadol compound oral solution in accordance with the present comprises a flavoring agent to mask the bitter tastes of acetaminophen and tramadol, the acetaminophen and tramadol keep exist as co-solutes and thus the oral solution formulation remain stable.

The term "tramadol" as used herein, unless otherwise described, is drawn to "tramadol" and "tramadol hydrochloride."

### Example 1

The instant example relates to defining the dissolving of acetaminophen and tramadol co-existing in the acetaminophen and tramadol compound oral solution. An acetaminophen and tramadol compound oral solution is defined to have the acetaminophen and tramadol co-existing therein dissolved where the solution apprears clear and transparent to visual observation with the naked eye. Each formulation is prepared in a transparent centrifugation tube and stood for 24 hours before visual observation. A solution in a centrifugation tube with neither visible precipitation nor visible suspensoid at the bottom of the centrifugation tube is defined as a clear and transparent solution.

Should precipitation particles or cotton-like suspensoids found visible at the bottom of a centrifugation tube, the solution therein is defined as a solution not having the co-existing acetaminophen and tramadol dissolved.

Where a clear and transparent solution freshly prepared based on a formulation produces crystalloid precipitation after being stood for 24 hours, the solution is defined as a recrystalizing solution. In order to further confirm the existence of persisting crystalloid precipitation, the solution may be ultrasonically shaked for 30 minutes to dissolve the crystalloid, after which visual observation is performed to determine the recrystalizing characteristic of the solution.

Specifically, each formulation is prepared as a solution in a transparent centrifugation tube. The tube containing the solution is then stood for 24 hours. Visual observation is performed to examine the bottom of the tube.

The method as described in the instant example applies when determine if solutions in following examples are clear and transparent solutions.

### Example 2

The instant example relates to the production of acetaminophen and tramadol compound oral solutions and determination on whether the solutions are clear and transparent. In the instant example, the solvent system comprises glycerin and water as primary ingredients, and propylene glycol and various polyethylene glycols as assisting co-solvents. The polyethylene glycols employed in the instant example are PEG400, PEG1000, and PEG3350. Water and glycerin and/or propylene glycol are first mixed to form a homogeneously mixed solution, to which acetaminophen, tramadol and other ingredients are added. Ultrasonic shaking is performed to facilitate dissolving of the ingredients. In the instant example, the solution is examined with the method as described in example 1 to determine if the solution is a clear and transparent solution. Formulations 37, and 39 to 43 are described as follows:

| Formulation 37 | Weight | Weight Percentage |
|---|---|---|
| Acetaminophen | 1625.00 mg | 5.64% |
| Tramadol hydrochloride | 187.50 mg | 0.65% |
| Glycerin | 6305.00 mg | 21.87% |
| Propylene glycol | 10360.00 mg | 35.94% |
| Water | 10000.00 mg | 34.69% |
| Sucralose | 231.00 mg | 0.80% |
| Pineapple flavor | 115.00 mg | 0.40% |
| Yellow No. 4 | 5.80 mg | 0.02% |
| Total | 28829.30 mg | 100.00% |

| Formulation 39 | Weight | Weight Percentage |
|---|---|---|
| Acetaminophen | 1625.00 mg | 5.62% |
| Tramadol hydrochloride | 187.50 mg | 0.65% |
| Glycerin | 12610.00 mg | 43.61% |
| Propylene glycol | 3626.00 mg | 12.54% |
| PEG400 | 6768.00 mg | 23.40% |
| Water | 3750.00 mg | 12.97% |
| Sucralose | 231.00 mg | 0.80% |
| Pineapple flavor | 115.00 mg | 0.40% |
| Yellow No. 4 | 5.80 mg | 0.02% |
| Total | 28918.30 mg | 100.00% |

| Formulation 40 | Weight | Weight Percentage |
|---|---|---|
| Acetaminophen | 1625.00 mg | 5.46% |
| Tramadol hydrochloride | 187.50 mg | 0.63% |
| Glycerin | 14701.70 mg | 49.42% |
| Propylene glycol | 10152.00 mg | 34.12% |
| PEG400 | 2732.00 mg | 9.18% |
| Water | 231.00 mg | 0.78% |
| Sucralose | 115.00 mg | 0.39% |
| Pineapple flavor | 5.80 mg | 0.02% |
| Yellow No. 4 | 29750.00 mg | 100.00% |
| Total | | |

| Formulation 41 | Weight | Weight Percentage |
|---|---|---|
| Acetaminophen | 1625.00 mg | 5.52% |
| Tramadol hydrochloride | 187.50 mg | 0.64% |
| Glycerin | 15762.50 mg | 53.57% |
| PEG 1000 | 7500.00 mg | 25.49% |
| Water | 4000.00 mg | 13.59% |
| Sucralose | 231.00 mg | 0.78% |
| Pineapple flavor | 115.00 mg | 0.39% |
| Yellow No. 4 | 5.80 mg | 0.02% |
| Total | 29426.80 mg | 100.00% |

| Formulation 42 | Weight | Weight Percentage |
|---|---|---|
| Acetaminophen | 1625.00 mg | 5.59% |
| Tramadol hydrochloride | 187.50 mg | 0.64% |
| Glycerin | 16383.70 mg | 56.32% |
| PEG3350 | 5076.00 mg | 17.45% |
| Water | 5464.00 mg | 18.78% |
| Sucralose | 231.00 mg | 0.79% |
| Pineapple flavor | 115.00 mg | 0.40% |
| Yellow No. 4 | 5.80 mg | 0.02% |
| Total | 29088.00 mg | 100.00% |

| Formulation 43 | Weight | Weight Percentage |
|---|---|---|
| Acetaminophen | 1625.00 mg | 5.55% |
| Tramadol hydrochloride | 187.50 mg | 0.64% |
| Glycerin | 14045.70 mg | 47.96% |
| PEG3350 | 7614.00 mg | 26.00% |
| Water | 5464.00 mg | 18.66% |
| Sucralose | 231.00 mg | 0.79% |
| Pineapple flavor | 115.00 mg | 0.39% |
| Yellow No. 4 | 5.80 mg | 0.02% |
| Total | 29288.00 mg | 100.00% |

Formulations 37, 39 to 43 are determined with the method as described in Example 1 as clear and transparent solutions.

Formulations 37, 39 to 43 are sorted according to the ratios of PEG400, PEG1000, and PEG3350 as follows, where symbol "O" is designated to a solution determined as a "clear and transparent solution," while symbol "X" is designated to a solution failed to be determined as a "clear and transparent solution."

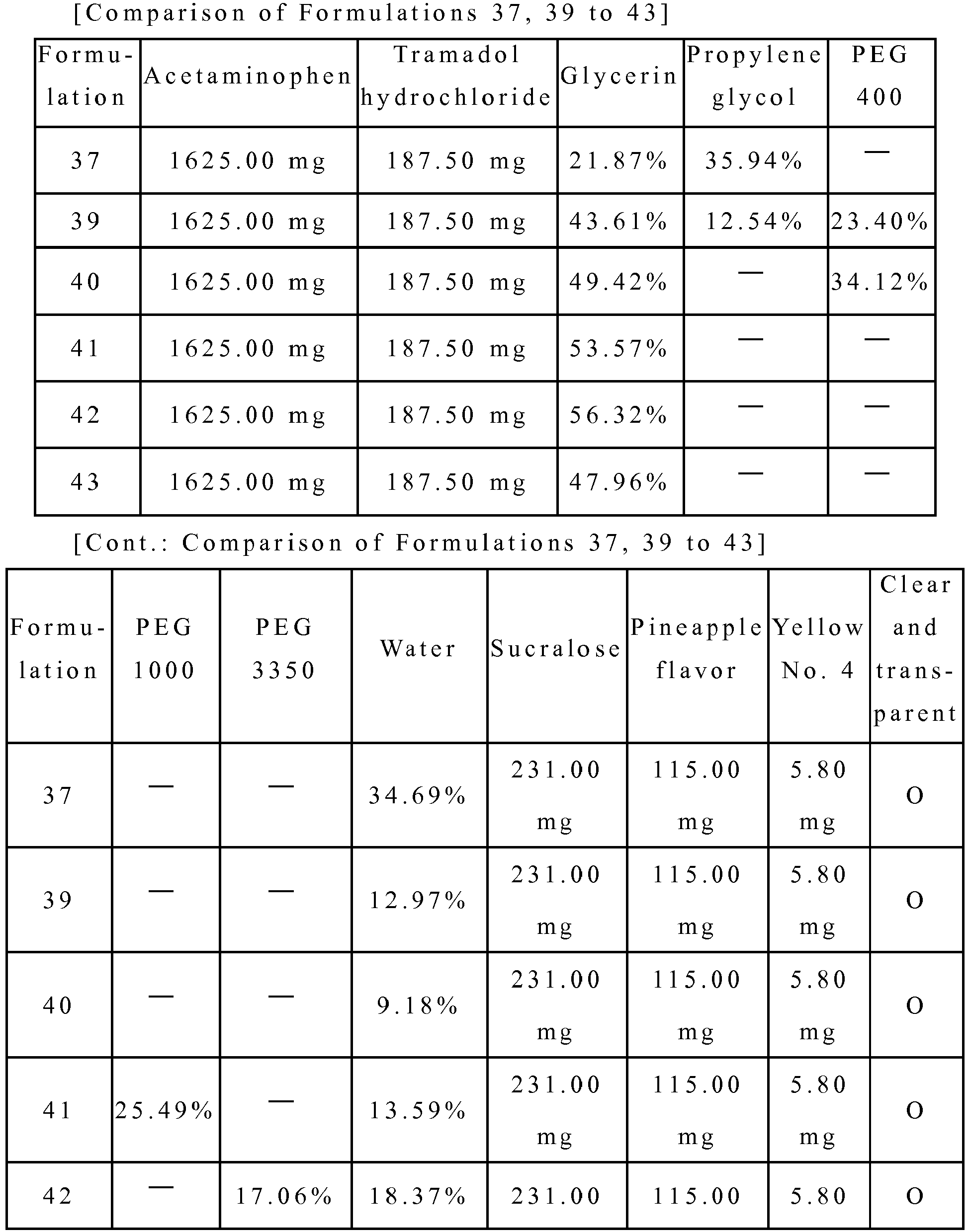

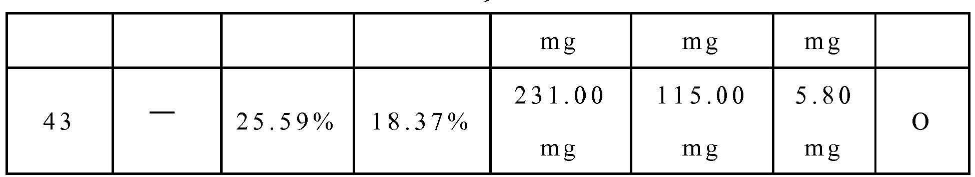

In the instant example the weight percentage of the solvent system is 92 wt% to 93 wt%. In the instant example the formulations employing a solvent system comprising glycerin, propylene glycol, water, and polyethylene glycol, the weight percentage of polyethylene glycol is from 13.50 wt% to 23.40 wt%, or approximately 13 wt% to 24 wt%, wherein the polyethylene glycol used in such formulations is PEG400. In the instant example the formulations employing a solvent system comprising glycerin, water, and polyethylene glycol, the weight percentage of polyethylene glycol is from 17.06 wt% to 34.12 wt%, or approximately 17 wt% to 35 wt%.

In a solvent system comprising glycerin, water, and polyethylene glycol, wherein the polyethylene glycol used in such formulations is PEG400, the weight percentage of the PEG400 is 34.12 wt%, or approximately 34 wt% to 35 wt%; wherein the polyethylene glycol used in such formulations is PEG1000, the weight percentage of the PEG1000 is 25.49 wt%, or approximately 25 wt% to 26 wt%; wherein the polyethylene glycol used in such formulations is PEG3350, the weight percentage of the PEG3350 is 17.06 wt% to 25.59 wt%, or approximately 17 wt% to 26 wt%.

Based on the aforementioned facts, the solvent system employed in the instant example having glycerin, propylene glycol, water, and polyethylene glycol, as demonstrated with Formulations 39 to 43, wherein the weight percentage of polyethylene glycol is 17 wt% to 24 wt%, dissolves the co-existing acetaminophen and tramadol. Preferrably, the solvent system employed in the instant example having glycerin, propylene glycol, water, and polyethylene glycol, as demonstrated with Formulations 39 to 43, wherein the weight percentage of polyethylene glycol is 23.40 wt%, dissolves the co-existing acetaminophen and tramadol.

The solvent systems employed in the instant example may have glycerin, water, and polyethylene glycol, using various polyethylene glycols. A solvent system containing 17 wt% to 35 wt% of PEG400, preferably 17.06 wt% to 34.12 wt%, allows co-existing acetaminophen and tramadol to be dissolved. A solvent system containing 25 wt% to 26 wt% of PEG1000, preferably 25.49 wt%, allows co-existing acetaminophen and tramadol to be dissolved. A solvent system containing 17 wt% to 26 wt% of PEG3350, preferably 17.06 wt% to 25.59 wt%, allows co-existing acetaminophen and tramadol to be dissolved.

### Example 3

The instant example relates to the production of an acetaminophen and tramadol compound oral solution and determination on whether the solution is clear and transparent. In the instant example, the solvent system contains glycerin and water as primary ingredients, and PEG400 an assisting co-solvent. Water and glycerin are first mixed to form a homogeneously mixed solution, to which acetaminophen, tramadol and other ingredients are gradually added. Ultrasonic shaking is performed to facilitate dissolving of the ingredients. In the instant example, the solution is examined with the method as described in example 1 to determine if the solution is a clear and transparent solution. Formulation 46 is described as follows:

| Formulation 46 | Weight | Weight percentage |
|---|---|---|
| Acetaminophen | 1625.00 mg | 5.45% |
| Tramadol hydrochloride | 187.50 mg | 0.63% |
| Glycerin | 14639.70 mg | 49.11% |
| PEG400 | 10152.00 mg | 34.06% |
| Water | 2794.00 mg | 9.37% |
| Sucralose | 231.00 mg | 0.77% |
| Pineapple flavor | 115.00 mg | 0.39% |
| Yellow No. 4 | 5.80 mg | 0.02% |

Formulation 46 is determined with the method as described in Example 1 as a clear and transparent solution.

Reviewing the solvent system comprising glycerin, water, and polyethylene glycol of the instant example with the method as described in Example 1, the weight percentage of polyethylene glycol is from 34.06 wt% to 34.12 wt%, or approximately 34 wt% to 35 wt%.

When further looking into the formulations of Examples 2 and 3, the solvent systems comprising glycerin, water, and polyethylene glycol, that the polyethylene glycol is PEG400 and that the weight percentage of PEG400 is 17 wt% to 35 wt%, preferably, 17.06 wt% to 34.12 wt%, dissolve the co-existing acetaminophen and tramadol.

### Example 4

The instant example relates to the production of acetaminophen and tramadol compound oral solutions, and determination on whether the acetaminophen and tramadol, as active pharmaceutical ingredients (API), are fully dissolved so that the solutions are clear and transparent. In the instant example, the formulations 38-1 to 38-4 are listed in the comparison table below, in which the weight unit is gram (g). The formulations listed are modifications to

Formulation 46 of Example 3 in aspects concerning API:

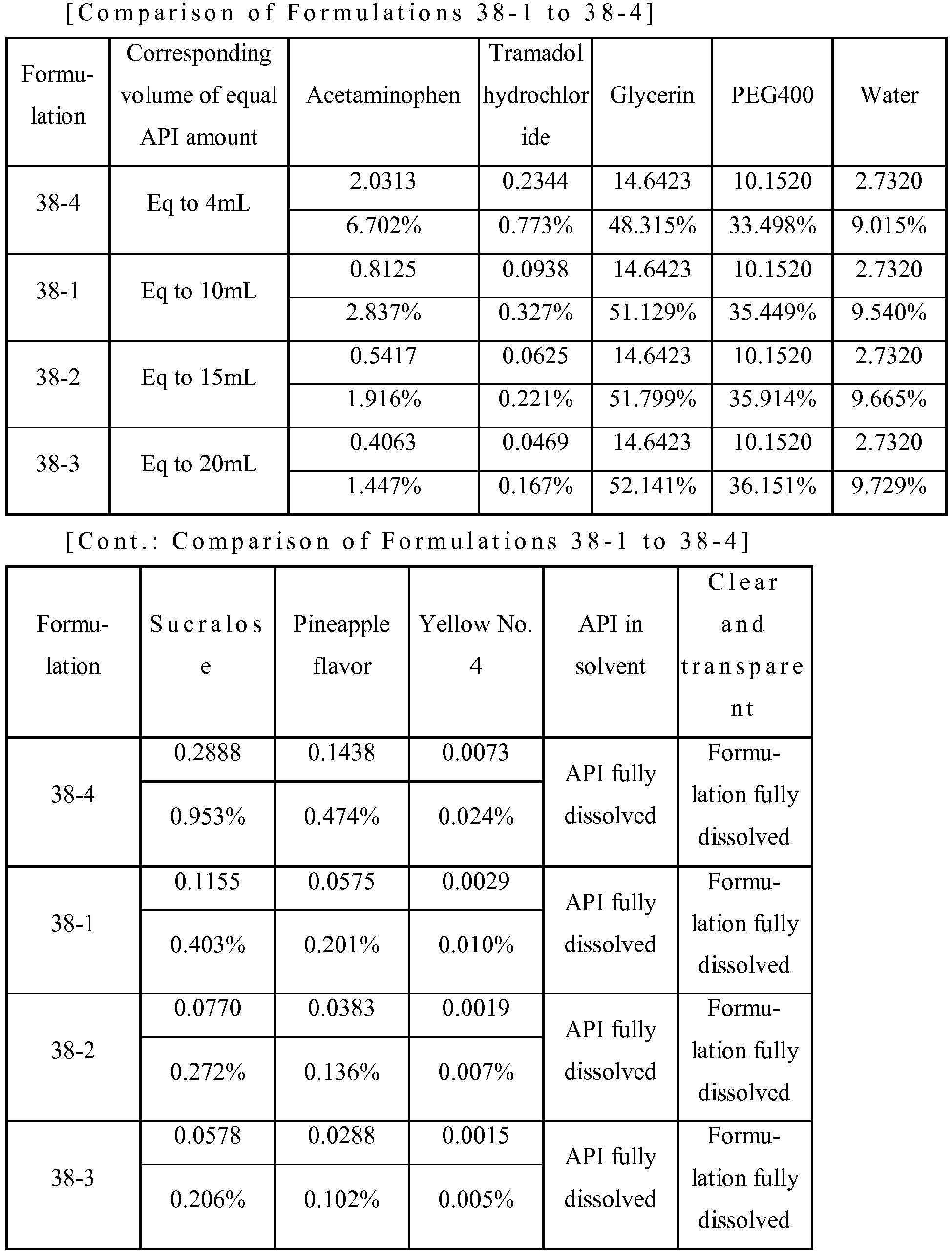

As shown with Formulations 38-1 to 38-4, when the ratio of a formulation is equal to dissolving 325 mg of acetaminophen and 37.5 mg of tramadol in a solvent system having a volume of 4 mL, 10 mL, 15 mL, or 20 mL, the formulation forms a solution whose ingredients are fully dissolved.

As a result, an acetaminophen and tramadol compound analgesic oral solution may be formed using a solvent system having glycerin, water, and polyethylene glycol, specifically that the polyethylene glycol is PEG400, and that the weight percentage of water is 3 wt% to 40 wt%, taking the total weight of the compound oral solution as 100%.

### Example 5

The instant example relates to the production of acetaminophen and tramadol compound oral solutions, and determination on whether the acetaminophen and tramadol, as API, are fully dissolved so that the solutions are clear and transparent. In the instant example, the formulations 38-6 to 38-9 are listed in the comparison table below, in which the weight unit is gram (g). The formulations listed are based on Formulation 46 of Example 3 that modifications are made to the weight percentage of glycerin and PEG400:

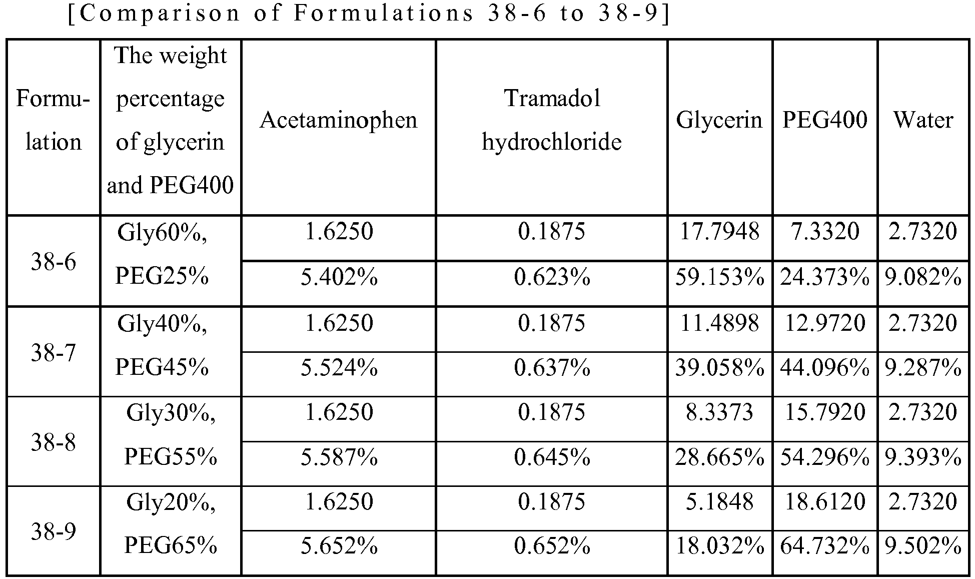

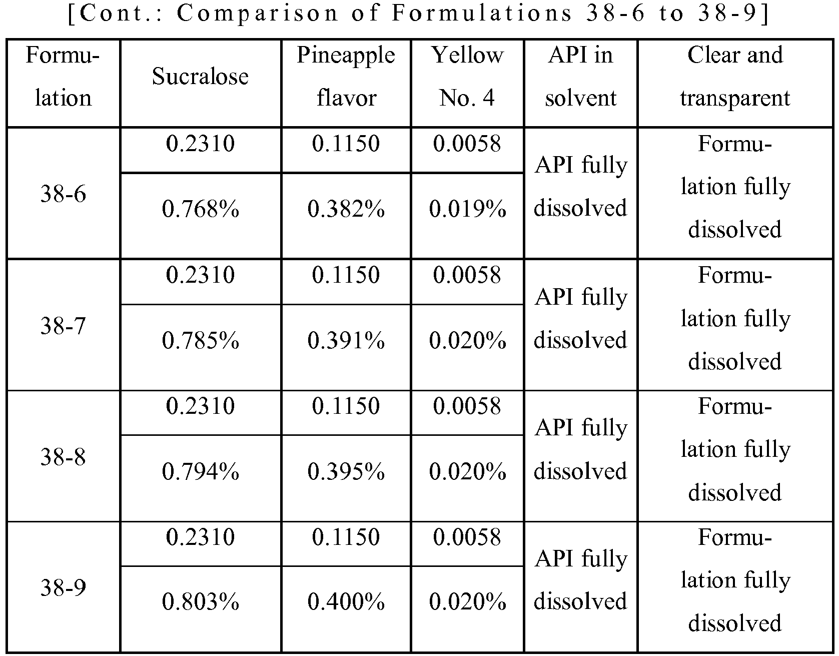

As shown with Formulations 38-6 to 38-9, when the ratio of the weight percentage of glycerin to the weight percentage of PEG400 is less than 2.4 (or approximately 2.5), the formulation forms a solution whose ingredients are fully dissolved. Thus, using higher ratio of PEG400 over glycerin helps dissolve the ingredients to form a clear and transparent solution. Higher ratio of PEG400, however, leads to an acetaminophen and tramadol compound analgestic oral solution of unfavored taste. Thus, the ratio of PEG400 may not be raised without a limit. In accordance with the formulations in the instant example, it is preferred that the ratio of the weight percentage of glycerin to the weight percentage of PEG400 is 0.3 to 2.4, with which a clear and transparent solution may be formed. In these formulations, specifically as shown in Formulation 38-9, eventhough the weight percentage of PEG400 is raised as high as 64.732 wt%, or approximately 65 wt%, a clear and transparent solution may still be formed.

As a result, an acetaminophen and tramadol compound analgesic oral solution may be formed using a solvent system having glycerin, water, and polyethylene glycol, specifically that the polyethylene glycol is PEG400, and that the ratio of the weight percentage of glycerin to the weight percentage of PEG400 is less than 2.5, preferably 0.3 to 2.4.

### Example 6

The instant example relates to the production of acetaminophen and tramadol compound oral solutions, and determination on whether the acetaminophen and tramadol, as API, are fully dissolved so that the solutions are clear and transparent. In the instant example, the formulations 38-10 to 38-14, 38-16 to 38-22 are listed in the comparison table below, in which the weight unit is gram (g). The formulations listed are based on Formulation 46 of Example 3 that the weight percentage of water is adjusted and that the weight percentages of glycerin and PEG400 are lowered with ratio thereof maintained:

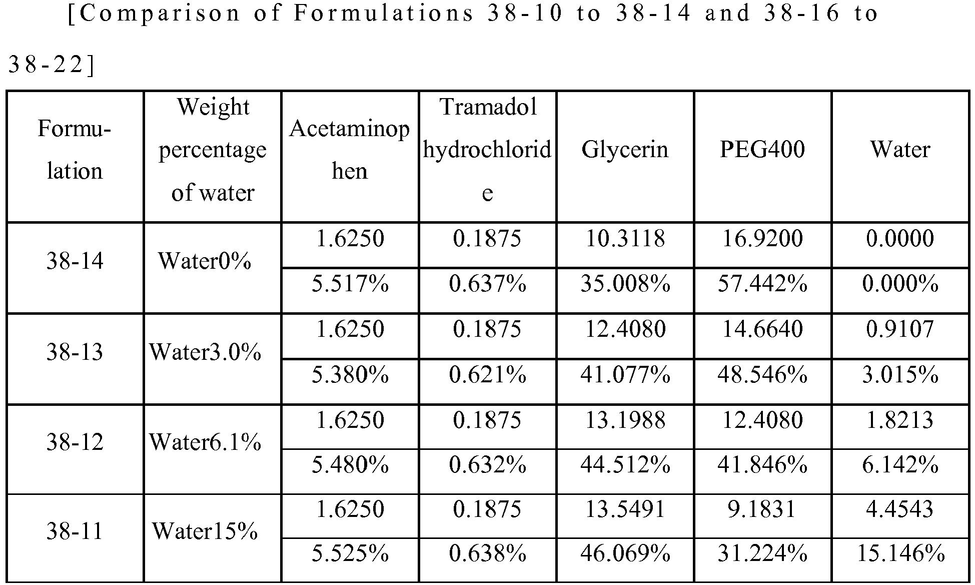

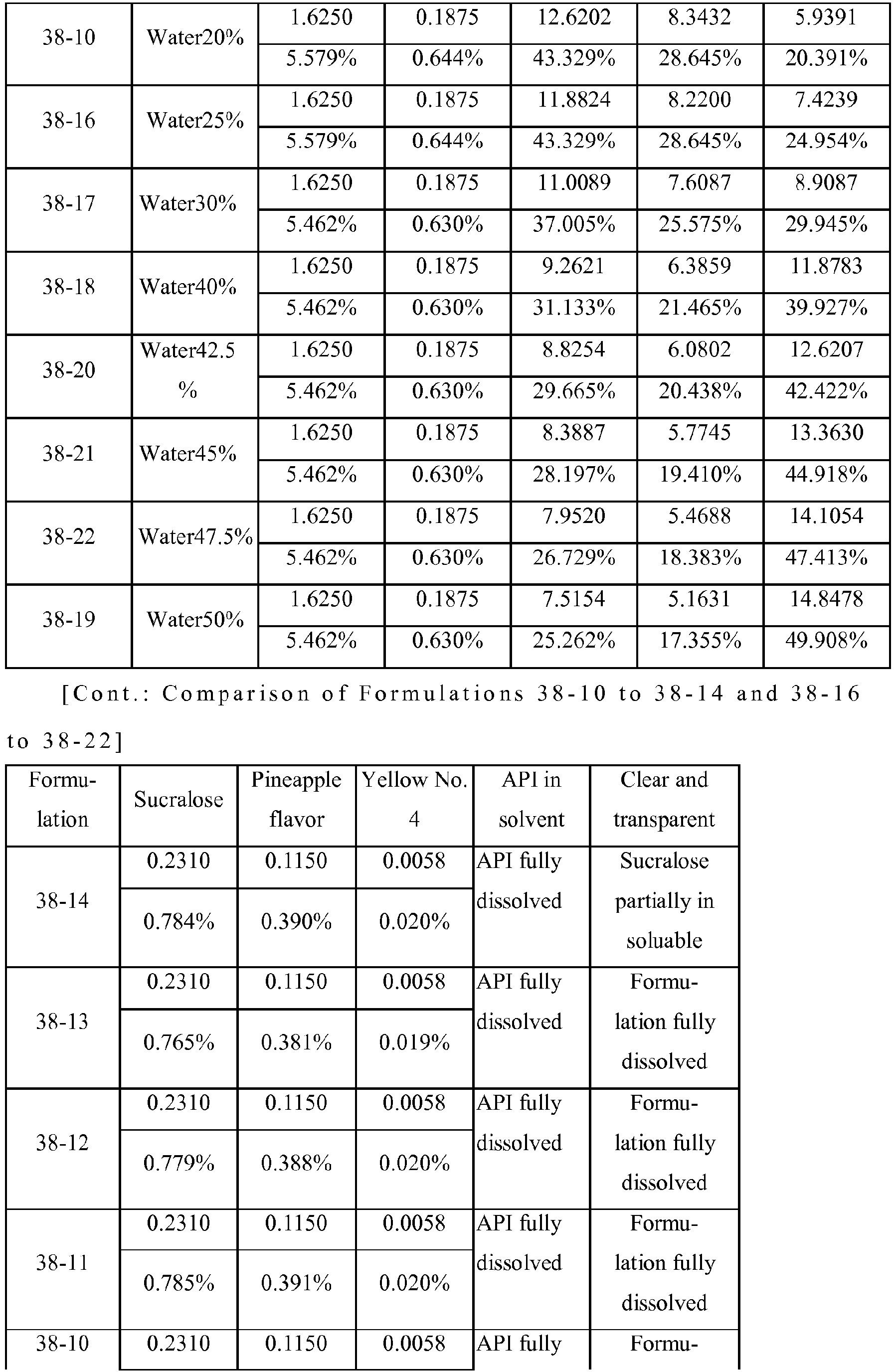

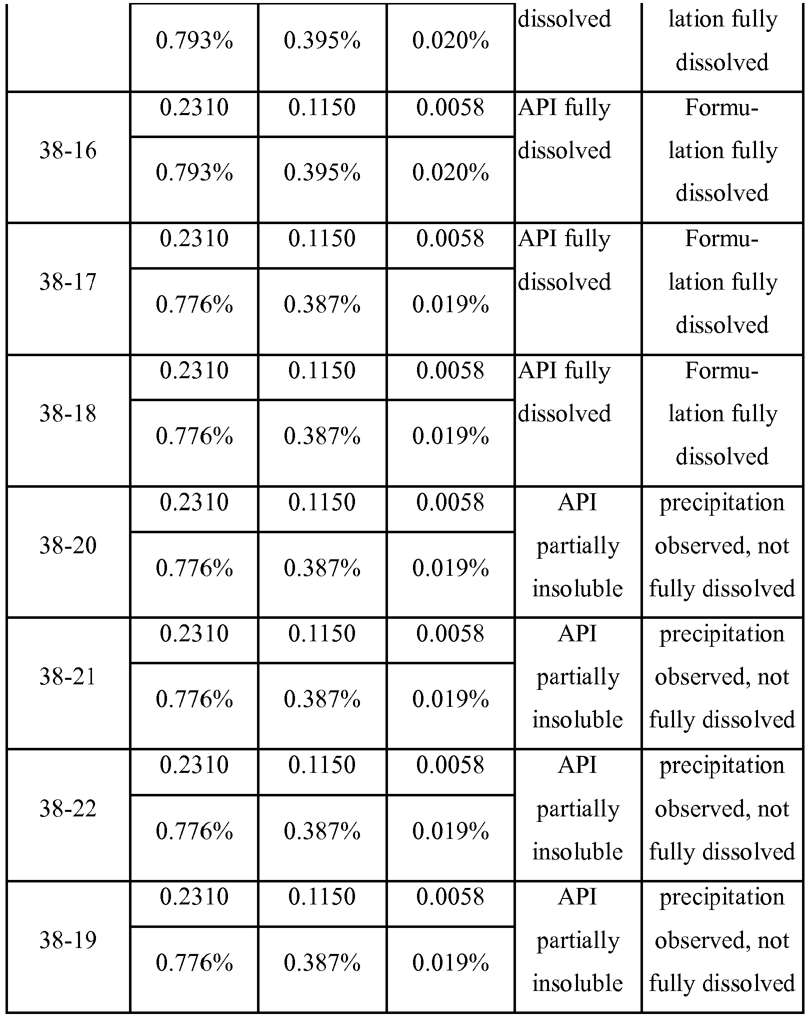

As demonstrated with Formulation 38-14, when the weight percentage of water is 0%, or no water included, API is dissolved rapidly. Other ingredients having been added, the water-soluble sucralose leads to partial precipitation and disrupts the formation of a clear and transparent solution.

Furthermore, as demonstrated with Formulations 38-19 to 38-22, when the weight percentage of water is higher than 42.5 wt%, API precipitation is observed. Other ingredients having been added and dissolved, the acetaminophen and tramadol, as API, partially recrystalize and disrupt the formation of a clear and transparent solution.

However, as demonstrated with Formulations 38-10 to 38-13 and 38-16 to 38-18, when the weight percentage of water is 3.0 wt% to 40 wt%, a clear and transparent solution may be formed.

Therefore, a solvent system having glycerin, water, and polyethylene glycol, specifically that the polyethylene glycol is PEG400, and that the weight percentage of water is 3.0 wt% to 40 wt%, a acetaminophen and tramadol compound analgesic oral solution may be formed.

Comparison of the formulations in Examples 4 to 6, it is concluded that when the dosage of API is maintained, the total volume of the oral solution may be 4 mL to 20 mL to allow the ingredients to be fully dissolved. When the total volume of the oral solution is less than 4 mL, insoluble precipitation is found in the solution.

When the weight percentage of glycerin higher than 70 wt%, it is difficult to have acetaminophen dissolved. Therefore precipitation is produced. The higher the weight percentage of PEG400 is, the tendancy of acetaminophen being dissolved is more obvious. Large amount of PEG400, however, leads to unfavored taste.

Furthermore, in the instant example, clear and transparent solutions are formed based on formulations with less than 40 wt% of water.

### Example 7

The instant example relates to the production of an acetaminophen and tramadol compound oral solution and determination on whether the solution is clear and transparent with the ingredients thereof fully dissolved. In the instant example, Formulation 39-1 as listed in the table below (in an amount of 5 mL; weight unit: mg) is based on Formulation 46 of Example 3 with different solvents or co-solvents:

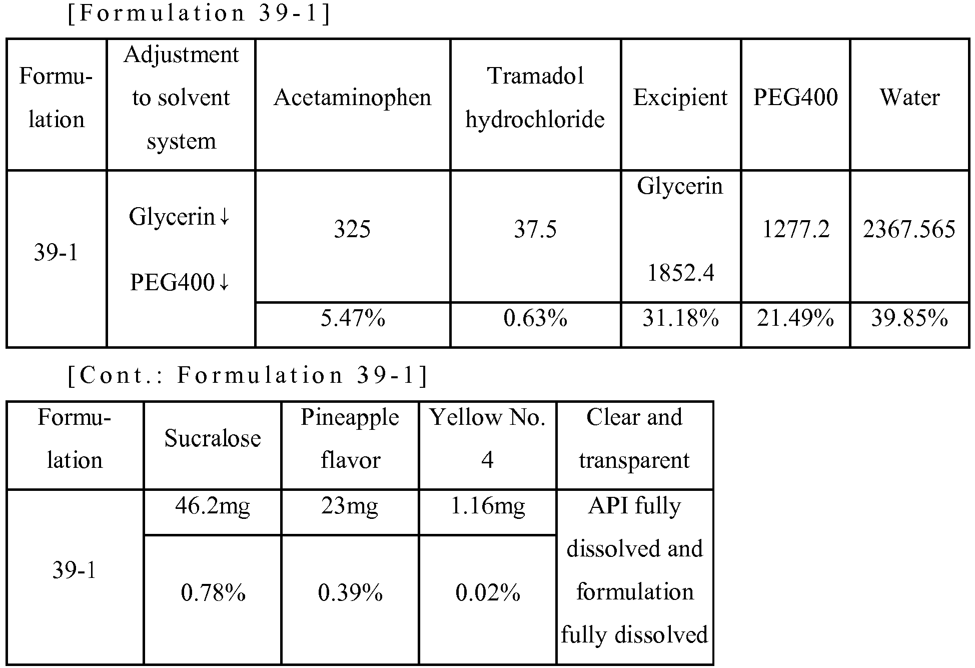

As demonstrated with Formulation 39-1, when the weight percentage of glycerin and the weight percentage of PEG400 are lowered, a clear and transparent solution may still be formed.

It is therefore reasonable to conclude that a solvent system employing PEG400 with glycerin and water helps form an acetaminophen and tramadol compound oral solution.

### Example 8

The instant example relates to formulations with adjustments to Formulation 46 of Example 3 in lowering the weight percentage of glycerin and the weight percentage of PEG400 for manufacturing of acetaminophen and tramadol compound oral solution, and determination on whether the solution is clear and transparent with the ingredients thereof fully dissolved.

In the instant example, Formulation 41-6 and 41-7 as listed in the table below (in an amount of 5 mL; weight unit: mg) are is based on Formulation 46 of Example 3 and comprise Yellow No. 4, sucralose and pineapple flavor:

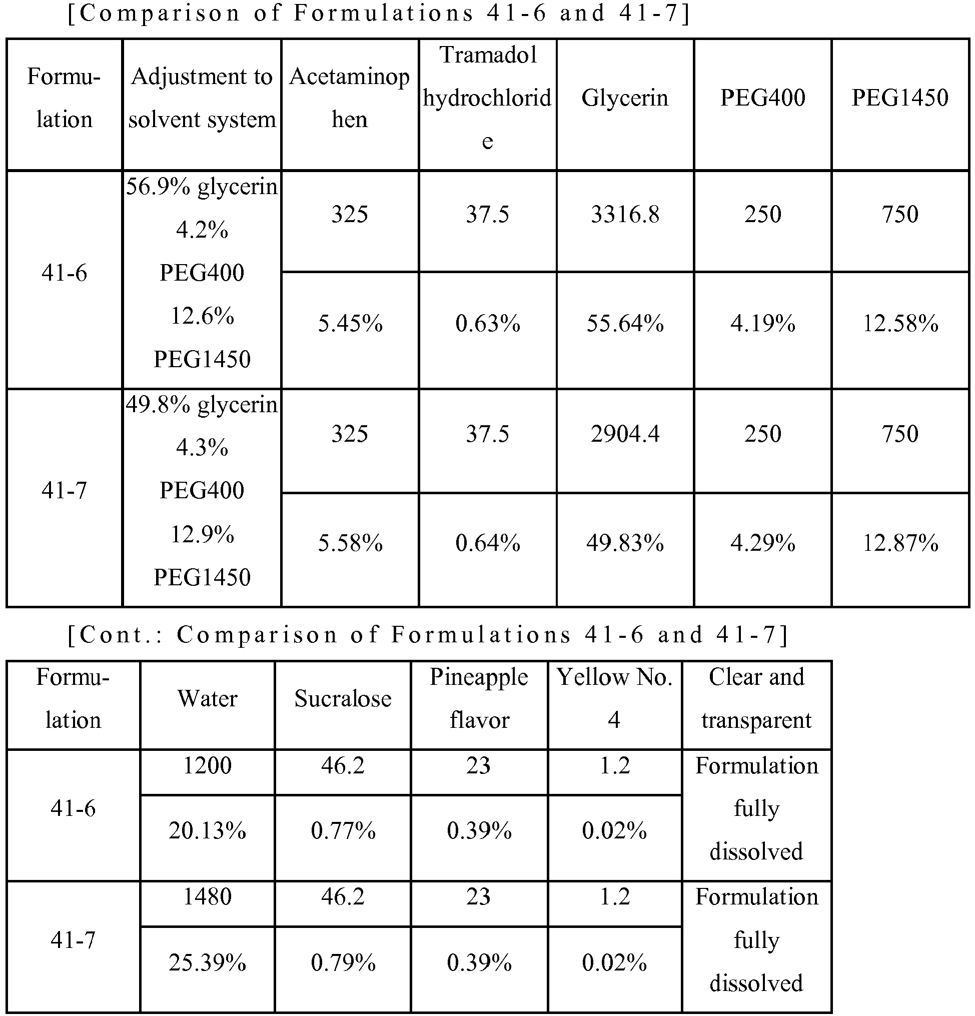

As demonstrated with Formulations 41-6 and 41-7, lowering the weight percentage of glycerin and the weight percentage of PEG400 and adding a suitable amount of PEG1450, allows the ingredients be dissolved and a clear and transparent solution be formed.

It is therefore understood that lowering the weight percentage of glycerin and the weight percentage of PEG400, and employing PEG1450, which is also a polyethylene glycol as PEG400, form an acetaminophen and tramadol compound oral solution, ingredients of which fully dissolved and no precipitation observed even after beening kept in cold storage.

### Example 9

The instant example relates to formulations replacing PEG400, as employed in Formulations 41-6 and 41-7, with propylene glycol for manufacturing acetaminophen and tramadol compound oral solutions, as well as determination on whether the solutions are clear and transparent with the ingredients thereof fully dissolved.

In the instant example, Formulations as listed in the table below (in an amount of 5 mL; weight unit: mg) are based on Formulations 41-6 and 41-7 of Example 8, with PEG400 be replaced with propylene glycol:

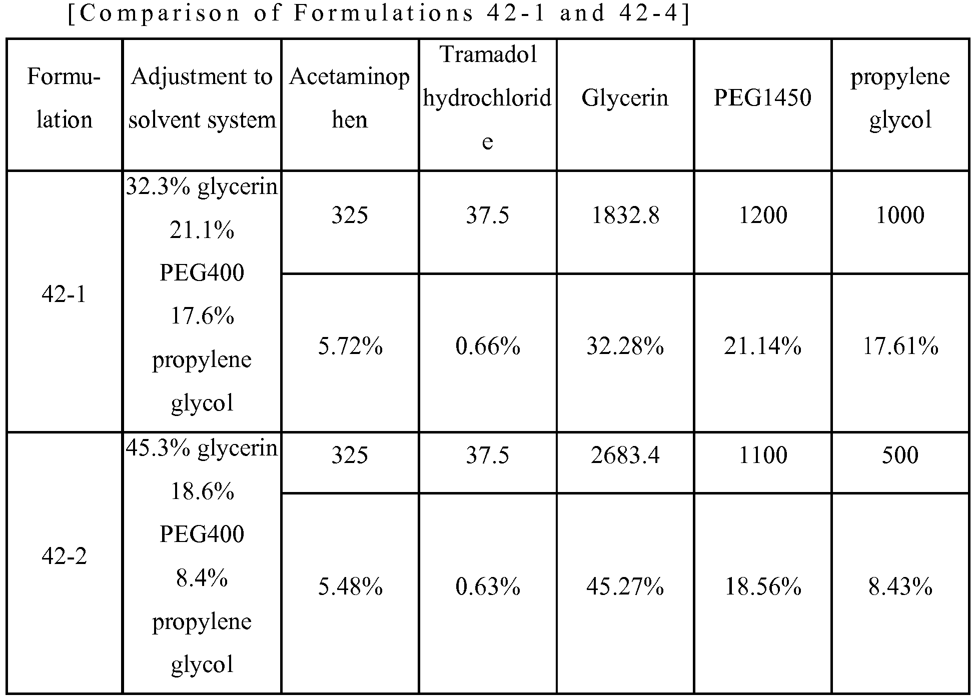

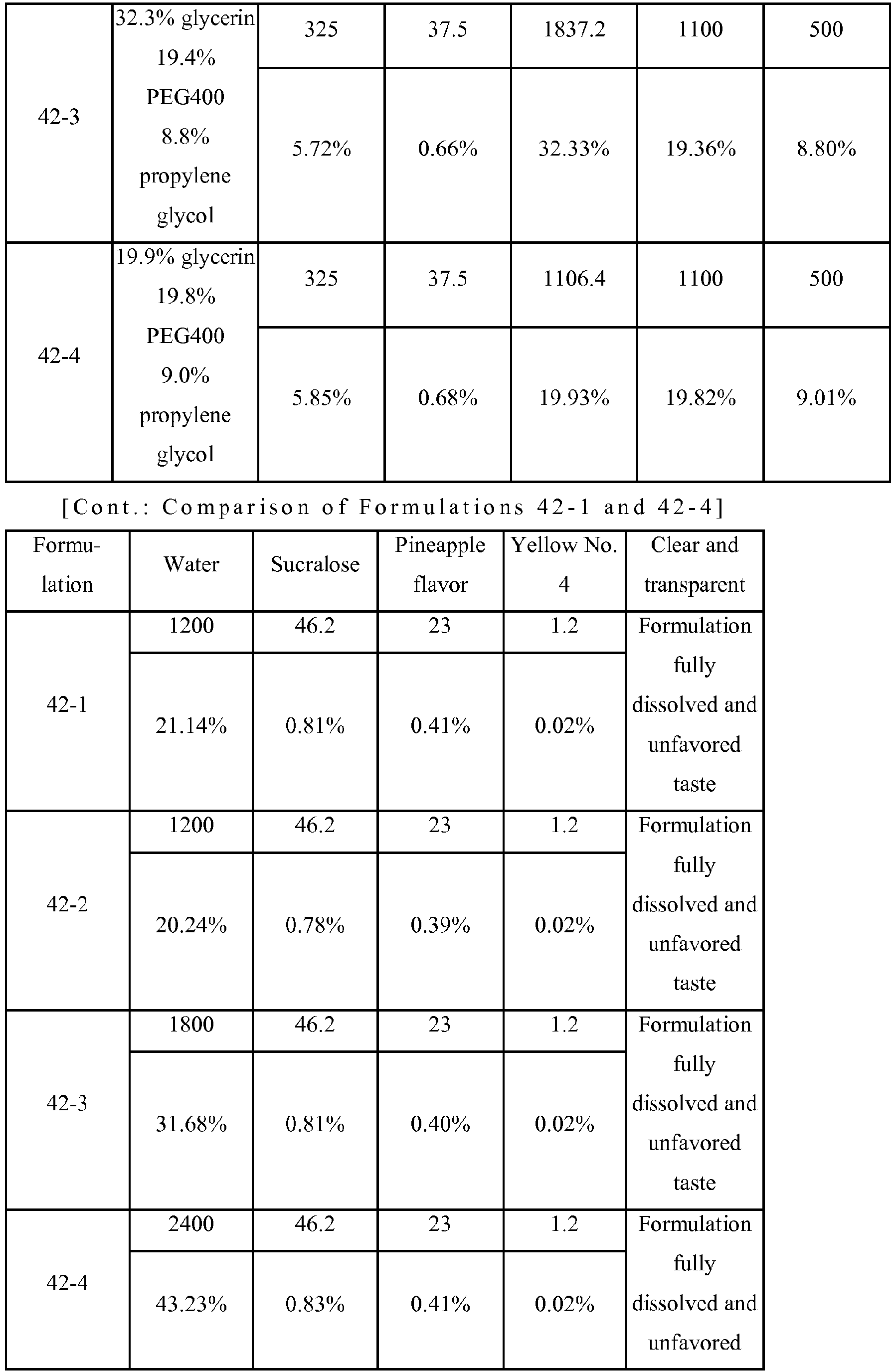

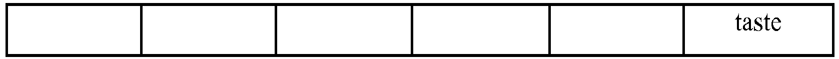

As demonstrated with Formulations 42-1 to 42-4, under the scheme of the formulations of the instant example, when the weight percentage of PEG1450 is approximately 20 wt%, a clear and transpartent acetaminophen and tramadol compound oral solution, ingredients of which fully dissolved, may be formed.

It is therefore understood that a solvent system containing glycerin, PEG1450 and propylene glycol, under specific ratio, forms an acetaminophen and tramadol compound oral solution, ingredients of which fully dissolved and no precipitation observed even after beening kept in cold storage.

### Example 10

The instant example relates to formulations for testing conditions with or without PEG400, which is employed in Formulations 41-6 and 41-7 of Example 8, for manufacturing acetaminophen and tramadol compound oral solution, as well as determination on whether the solution is clear and transparent with the ingredients thereof fully dissolved.

In the instant example, Formulations 42-5 to 42-11 as listed in the table below (in an amount of 5 mL; weight unit: mg) are based on Formulations 41-6 and 41-7 of Example 8:

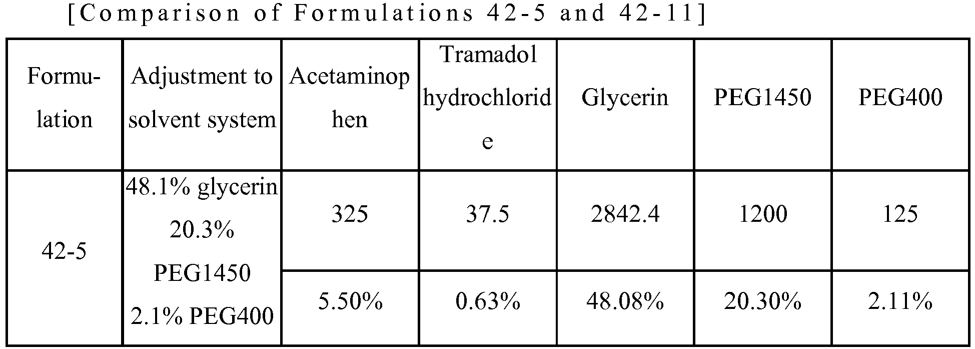

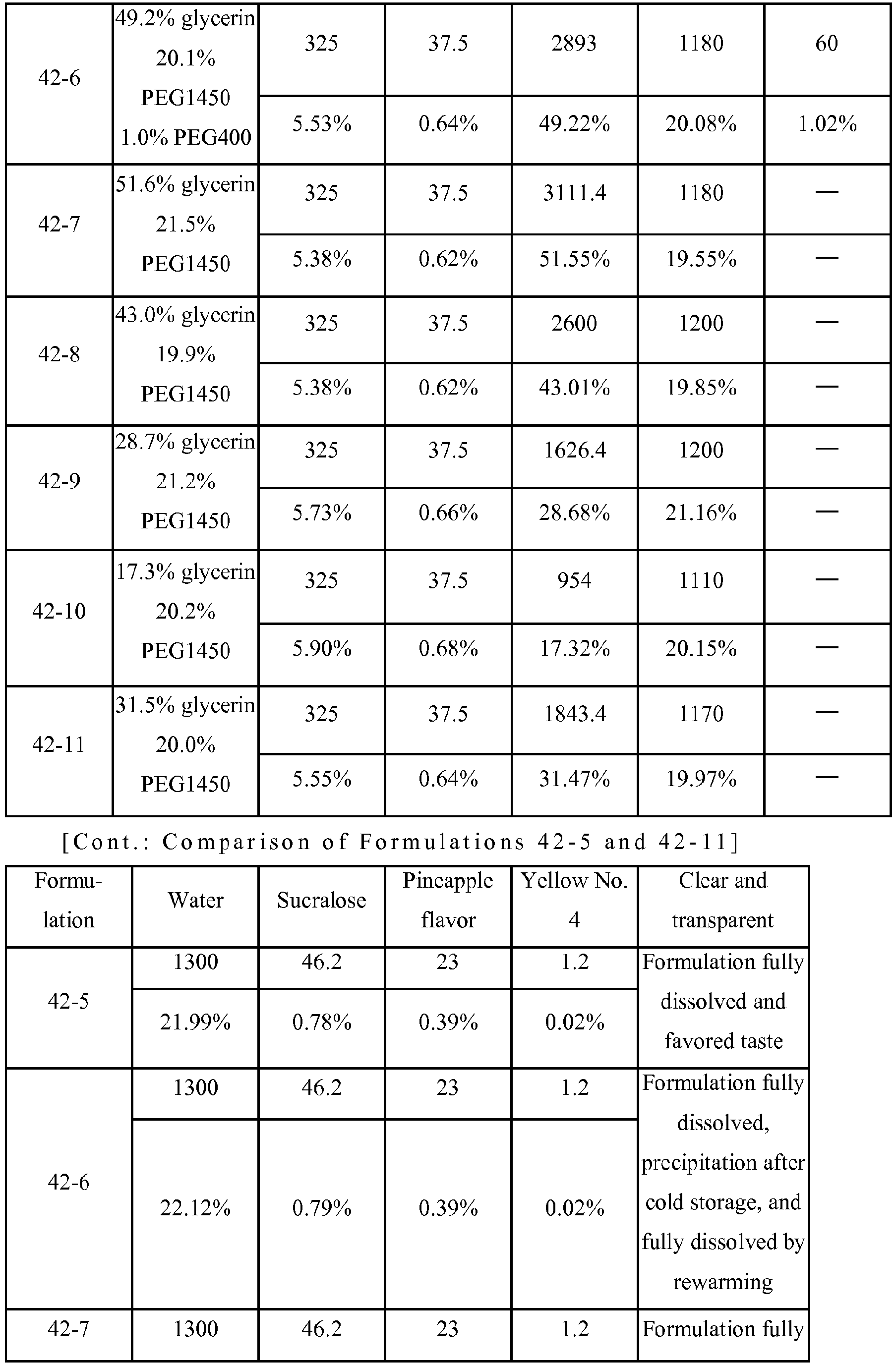

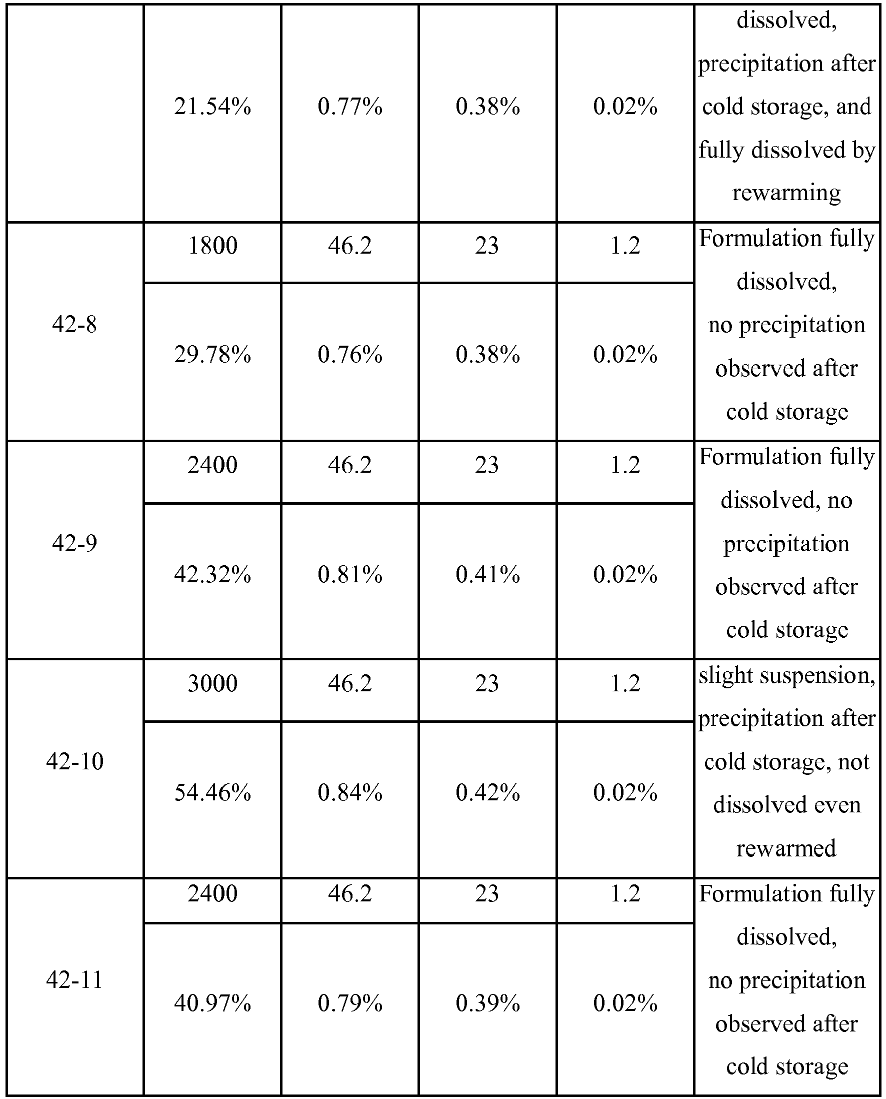

As demonstrated with Formulations 42-5 to 42-9, under the scheme of the formulations of the instant example, a solvent system having 20 wt% of PEG1450, accompanied with glycerin, ether further comprising PEG400 or not, is capable of forming a clear and transparent acetaminophen and tramadol compound oral solution with ingredients fully dissolved. As demonstrated with Formulations 42-6 to 42-9, in contrast to Formulation 42-10, since PEG1450 is solid at room temperature, a solution prepared based on a formulation containing PEG1450 freezes due to the characteristic of PEG1450. Further looking into Formulations 42-9 and 42-11, adding glycerin to a weight percentage higher than 28.7 wt% prevents said freezing.

Furthermore, as demonstrated with Formulation 42-6 and 42-7, water-soluble sucralose, in formulations with low water content, tends to produce crystalloid precipitation under low temperature, however, is dissolved after being rewarmed. Raising the weight percentage of water equal to or larger than 29.8 wt% prevents recrystallization of sucralose.

As demonstrated above, combining glycerin, PEG1450, and PEG400, or combining only glycerin and PEG1450 (e.g.: using 31.47 wt% of glycerin, 19.97 wt% of PEG1450, and 40.97 wt% of water as in Formulation 42-11), under specific ratio, forms an acetaminophen and tramadol compound oral solution, ingredients of which fully dissolved and no precipitation observed even after beening kept in cold storage.

### Example 11

The instant example relates to formulations containing PEG1450 and PEG400 for manufacturing acetaminophen and tramadol compound oral solutions, as well as determination on whether the solution is clear and transparent with the ingredients thereof fully dissolved.

In the instant example, Formulations 50-1 to 50-10 as listed in the table below (in an amount of 5 mL; weight unit: mg) are implemented for producing acetaminophen and tramadol compound oral solutions:

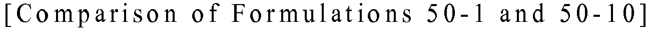

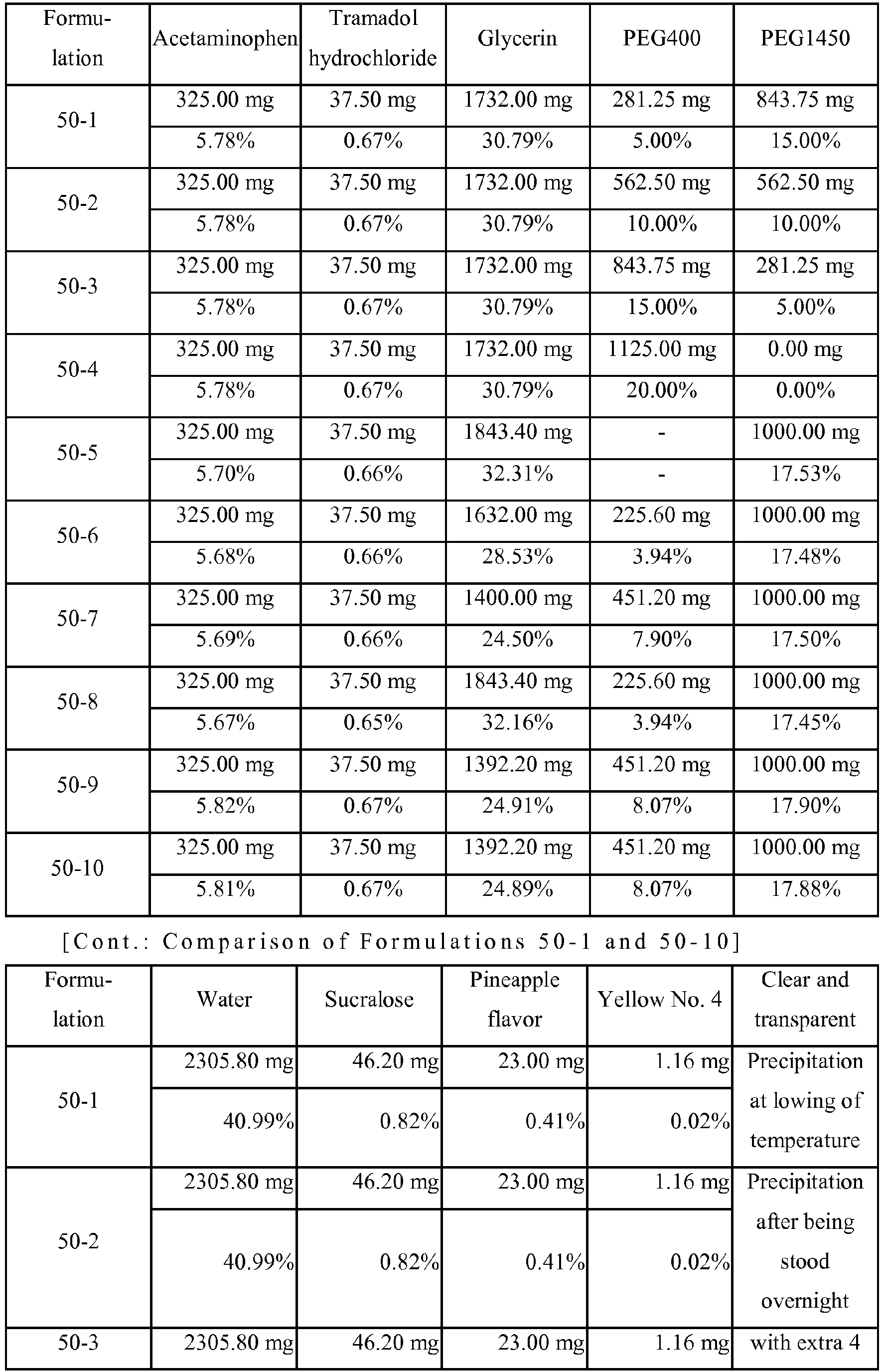

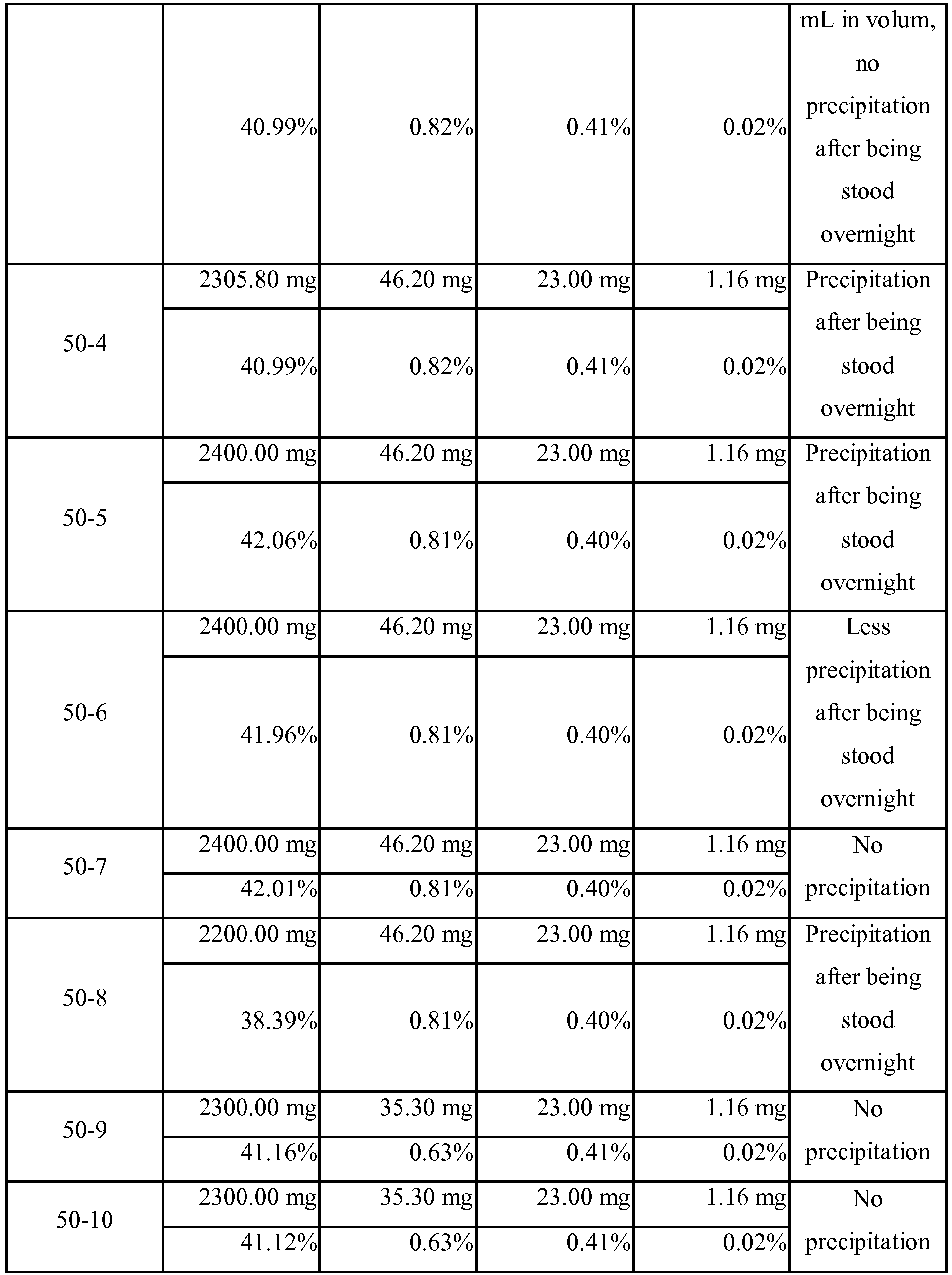

Comparing the formulations of the instant example, specifically Formulation 50-7, 50-9, 50-10, in conditions that the weight percentage of polyethylene glycol, being PEG1450 with PEG400, is 25 wt%, and that the weight percentage of glycerin is approximately equal to or less than 25 wt%, clear and transparent acetaminophen and tramadol compound oral solution forms, in which ingredients are fully dissolved.

Based on the above results, it is concluded that a solvent system comprising water, glycerin, PEG1450, and PEG400, under specific ratio, forms an acetaminophen and tramadol compound oral solution, ingredients of which fully dissolved and no precipitation observed even after beening kept in cold storage.

### Example 12

The instant example relates to formulations employing a single polyethylene glycol variant for manufacturing acetaminophen and tramadol compound oral solutions, as well as determination on whether the solution is clear and transparent with the ingredients thereof fully dissolved.

In the instant example, Formulations GP300_1 to GP300_24, GP400 1 to GP400 24, GP1450_1 to GP1450_24, and GP3350_1 to GP3350-24 as listed in the table below (in an amount of 10 mL) are implemented for producing acetaminophen and tramadol compound oral solutions:

**[Comparison of Formulations GP300_1 to GP300_24]**

| Formulation | Acetaminophen | Tramadol hydrochloride | Glycerin | PEG300 | Water | Sucralose | Clear and transparent |
|---|---|---|---|---|---|---|---|
| GP300_1 | 650.00 mg | 75.00 mg | 500.00 mg | 2500.00 mg | 6830.32 mg | 102.76 mg | Formulation fully dissolved, precipitation after cold storage |
| | 6.10% | 0.70% | 4.69% | 23.47% | 64.13 % | 0.96% | |
| GP300_2 | 650.00 mg | 75.00 mg | 1000.00 mg | 2000.00 mg | 6830.32 mg | 102.76 mg | Formulation fully dissolved, precipitation after cold storage |
| | 6.10% | 0.70% | 9.39% | 18.78% | 64.13% | 0.96% | |
| GP300_3 | 650.00 mg | 75.00 mg | 1500.00 mg | 1500.00 mg | 6830.32 mg | 102.76 mg | Formulation fully dissolved, precipitation after cold storage |
| | 6.10% | 0.70% | 14.08% | 14.08% | 64.13% | 0.96% | |
| GP300_4 | 650.00 mg | 75.00 mg | 500.00 mg | 3000.00 mg | 6342.44 mg | 95.42 mg | Formulation fully dissolved, precipitation after cold storage |
| | 6.10% | 0.70% | 4.69% | 28.17% | 59.55% | 0.90% | |
| GP300_5 | 650.00 mg | 75.00 mg | 1000.00 mg | 2500.00 mg | 6342.44 mg | 95.42 mg | Formulation fully dissolved, precipitation after cold storage |
| | 6.10% | 0.70% | 9.39% | 23.47% | 59.55% | 0.90% | |
| GP300_6 | 650.00 mg | 75.00 mg | 1500.00 mg | 2000.00 mg | 6342.44 mg | 95.42 mg | Formulation fully dissolved, precipitation after cold storage |
| | 6.10% | 0.70% | 14.08% | 18.78% | 59.55% | 0.90% | |
| GP300_7 | 650.00 mg | 75.00 mg | 2000.00 mg | 1500.00 mg | 6342.44 mg | 95.42 mg | Formulation fully dissolved, precipitation after cold storage |
| | 6.10% | 0.70% | 18.78% | 14.08% | 59.55% | 0.90% | |
| GP300_8 | 650.00 mg | 75.00 mg | 500.00 mg | 3500.00 mg | 5854.56 mg | 88.08 mg | No precipitation observed after cold storage |
| | 6.10% | 0.70% | 4.69% | 32.86% | 54.97% | 0.83% | |
| GP300_9 | 650.00 mg | 75.00 mg | 1000.00 mg | 3000.00 mg | 5854.56 mg | 88.08 mg | Formulation fully dissolved, precipitation after cold storage |
| | 6.10% | 0.70% | 9.39% | 28.17% | 54.97% | 0.83% | |
| GP300_10 | 650.00 mg | 75.00 mg | 2000.00 mg | 2000.00 mg | 5854.56 mg | 88.08 mg | Formulation fully dissolved, precipitation after cold storage |
| | 6.10% | 0.70% | 18.78% | 18.78% | 54.97% | 0.83% | |
| GP300_11 | 650.00 mg | 75.00 mg | 2500.00 mg | 1500.00 mg | 5854.56 mg | 88.08 mg | Formulation fully dissolved, precipitation after cold storage |
| | 6.10% | 0.70% | 23.47% | 14.08% | 54.97% | 0.83% | |
| GP300_12 | 650.00 mg | 75.00 mg | 1000.00 mg | 3500.00 mg | 5366.68 mg | 80.74 mg | No precipitation observed after cold storage |
| | 6.10% | 0.70% | 9.39% | 32.86% | 50.39% | 0.76% | |
| GP300_13 | 650.00 mg | 75.00 mg | 1500.00 mg | 3000.00 mg | 5366.68 mg | 80.74 mg | Formulation fully dissolved, precipitation after cold storage |
| | 6.10% | 0.70% | 14.08% | 28.17% | 50.39% | 0.76% | |
| GP300_14 | 650.00 mg | 75.00 mg | 1500.00 mg | 3500.00 mg | 4878.80 mg | 73.40 mg | No precipitation observed after cold storage |
| | 6.10% | 0.70% | 14.08% | 32.86% | 45.81% | 0.69% | |
| GP300_15 | 650.00 mg | 75.00 mg | 2000.00 mg | 3000.00 mg | 4878.80 mg | 73.40 mg | No precipitation observed after cold storage |
| | 6.10% | 0.70% | 18.78% | 28.17% | 45.81% | 0.69% | |
| GP300_16 | 650.00 mg | 75.00 mg | 2500.00 mg | 2500.00 mg | 4878.80 mg | 73.40 mg | Formulation fully dissolved, precipitation after cold storage |
| | 6.10% | 0.70% | 23.47% | 23.47% | 45.81 % | 0.69% | |
| GP300_17 | 650.00 mg | 75.00 mg | 3000.00 mg | 2000.00 mg | 4878.80 mg | 73.40 mg | Formulation fully dissolved, precipitation after cold storage |
| | 6.10% | 0.70% | 28.17% | 18.78% | 45.81 % | 0.69% | |
| GP300_18 | 650.00 mg | 75.00 mg | 3500.00 mg | 1500.00 mg | 4878.80 mg | 73.40 mg | Formulation fully dissolved, precipitation after cold storage |
| | 6.10% | 0.70% | 32.86% | 14.08% | 45.81% | 0.69% | |
| GP300_19 | 650.00 mg | 75.00 mg | 2000.00 mg | 3500.00 mg | 4390.92 mg | 66.06 mg | No precipitation observed after cold storage |
| | 6.10% | 0.70% | 18.78% | 32.86% | 41.23% | 0.62% | |
| GP300_20 | 650.00 mg | 75.00 mg | 2500.00 mg | 3000.00 mg | 4390.92 mg | 66.06 mg | Formulation fully dissolved, precipitation after cold storage |
| | 6.10% | 0.70% | 23.47% | 28.17% | 41.23% | 0.62% | |
| GP300_21 | 650.00 mg | 75.00 mg | 3000.00 mg | 3000.00 mg | 3903.04 mg | 58.72 mg | No precipitation observed after cold storage |
| | 6.10% | 0.70% | 28.17% | 28.17% | 36.65% | 0.55% | |
| GP300_22 | 650.00 mg | 75.00 mg | 3500.00 mg | 2500.00 mg | 3903.04 mg | 58.72 mg | Formulation fully dissolved, precipitation after cold storage |
| | 6.10% | 0.70% | 32.86% | 23.47% | 36.65% | 0.55% | |
| GP300_23 | 650.00 mg | 75.00 mg | 4000.00 mg | 2000.00 mg | 3903.04 mg | 58.72 mg | Formulation fully dissolved, precipitation after cold storage |
| | 6.10% | 0.70% | 37.56% | 18.78% | 36.65% | 0.55% | |
| GP300_24 | 650.00 mg | 75.00 mg | 4500.00 mg | 1500.00 mg | 3903.04 mg | 58.72 mg | Formulation fully dissolved, precipitation after cold storage |
| | 6.10% | 0.70% | 42.25% | 14.08% | 36.65% | 0.55% | |

**[Comparison of Formulations GP400_1 to GP400_24]**

| Formulation | Acetaminophen | Tramadol hydrochloride | Glycerin | PEG400 | Water | Sucralose | Clear and transparent |
|---|---|---|---|---|---|---|---|
| GP400_1 | 650.00 mg | 75.00 mg | 500.00 mg | 2500.00 mg | 6830.32 mg | 102.76 mg | Formulation fully dissolved, precipitation after cold storage |
| | 6.10% | 0.70% | 4.69% | 23.47% | 64.13% | 0.96% | |
| GP400_2 | 650.00 mg | 75.00 mg | 1000.00 mg | 2000.00 mg | 6830.32 mg | 102.76 mg | Formulation fully dissolved, precipitation after cold storage |
| | 6.10% | 0.70% | 9.39% | 18.78% | 64.13% | 0.96% | |
| GP400_3 | 650.00 mg | 75.00 mg | 1500.00 mg | 1500.00 mg | 6830.32 mg | 102.76 mg | Formulation fully dissolved, precipitation after cold storage |
| | 6.10% | 0.70% | 14.08% | 14.08% | 64.13% | 0.96% | |
| GP400_4 | 650.00 mg | 75.00 mg | 500.00 mg | 3000.00 mg | 6342.44 mg | 95.42 mg | Formulation fully dissolved, precipitation after cold storage |
| | 6.10% | 0.70% | 4.69% | 28.17% | 59.55% | 0.90% | |
| GP400_5 | 650.00 mg | 75.00 mg | 1000.00 mg | 2500.00 mg | 6342.44 mg | 95.42 mg | Formulation fully dissolved, precipitation after cold storage |
| | 6.10% | 0.70% | 9.39% | 23.47% | 59.55% | 0.90% | |
| GP400_6 | 650.00 mg | 75.00 mg | 1500.00 mg | 2000.00 mg | 6342.44 mg | 95.42 mg | Formulation fully dissolved, precipitation after cold storage |
| | 6.10% | 0.70% | 14.08% | 18.78% | 59.55% | 0.90% | |
| GP400_7 | 650.00 mg | 75.00 mg | 2000.00 mg | 1500.00 mg | 6342.44 mg | 95.42 mg | Formulation fully dissolved, precipitation after cold storage |
| | 6.10% | 0.70% | 18.78% | 14.08% | 59.55% | 0.90% | |
| GP400_8 | 650.00 mg | 75.00 mg | 500.00 mg | 3500.00 mg | 5854.56 mg | 88.08 mg | Formulation fully dissolved, precipitation after cold storage |
| | 6.10% | 0.70% | 4.69% | 32.86% | 54.97% | 0.83% | |
| GP400_9 | 650.00 mg | 75.00 mg | 1000.00 mg | 3000.00 mg | 5854.56 mg | 88.08 mg | Formulation fully dissolved, precipitation after cold storage |
| | 6.10% | 0.70% | 9.39% | 28.17% | 54.97% | 0.83% | |
| GP400_10 | 650.00 mg | 75.00 mg | 2000.00 mg | 2000.00 mg | 5854.56 mg | 88.08 mg | Formulation fully dissolved, precipitation after cold storage |
| | 6.10% | 0.70% | 18.78% | 18.78% | 54.97% | 0.83% | |
| GP400_11 | 650.00 mg | 75.00 mg | 2500.00 mg | 1500.00 mg | 5854.56 mg | 88.08 mg | Formulation fully dissolved, precipitation after cold storage |
| | 6.10% | 0.70% | 23.47% | 14.08% | 54.97% | 0.83% | |
| GP400_12 | 650.00 mg | 75.00 mg | 1000.00 mg | 3500.00 mg | 5366.68 mg | 80.74 mg | No precipitation observed after cold storage |
| | 6.10% | 0.70% | 9.39% | 32.86% | 50.39% | 0.76% | |
| GP400_13 | 650.00 mg | 75.00 mg | 1500.00 mg | 3000.00 mg | 5366.68 mg | 80.74 mg | No precipitation observed after cold storage |
| | 6.10% | 0.70% | 14.08% | 28.17% | 50.39% | 0.76% | |
| GP400_14 | 650.00 mg | 75.00 mg | 1500.00 mg | 3500.00 mg | 4878.80 mg | 73.40 mg | No precipitation observed after cold storage |
| | 6.10% | 0.70% | 14.08% | 32.86% | 45.81% | 0.69% | |
| GP400_15 | 650.00 mg | 75.00 mg | 2000.00 mg | 3000.00 mg | 4878.80 mg | 73.40 mg | No precipitation observed after cold storage |
| | 6.10% | 0.70% | 18.78% | 28.17% | 45.81% | 0.69% | |
| GP400_16 | 650.00 mg | 75.00 mg | 2500.00 mg | 2500.00 mg | 4878.80 mg | 73.40 mg | Formulation fully dissolved, precipitation after cold storage |
| | 6.10% | 0.70% | 23.47% | 23.47% | 45.81% | 0.69% | |
| GP400_17 | 650.00 mg | 75.00 mg | 3000.00 mg | 2000.00 mg | 4878.80 mg | 73.40 mg | Formulation fully dissolved, precipitation after cold storage |
| | 6.10% | 0.70% | 28.17% | 18.78% | 45.81% | 0.69% | |
| GP400_18 | 650.00 mg | 75.00 mg | 3500.00 mg | 1500.00 mg | 4878.80 mg | 73.40 mg | Formulation fully dissolved, precipitation after cold storage |
| | 6.10% | 0.70% | 32.86% | 14.08% | 45.81 % | 0.69% | |
| GP400_19 | 650.00 mg | 75.00 mg | 2000.00 mg | 3500.00 mg | 4390.92 mg | 66.06 mg | No precipitation observed after cold storage |
| | 6.10% | 0.70% | 18.78% | 32.86% | 41.23% | 0.62% | |
| GP400_20 | 650.00 mg | 75.00 mg | 2500.00 mg | 3000.00 mg | 4390.92 mg | 66.06 mg | No precipitation observed after cold storage |
| | 6.10% | 0.70% | 23.47% | 28.17% | 41.23% | 0.62% | |
| GP400_21 | 650.00 mg | 75.00 mg | 3000.00 mg | 3000.00 mg | 3903.04 mg | 58.72 mg | No precipitation observed after cold storage |
| | 6.10% | 0.70% | 28.17% | 28.17% | 36.65% | 0.55% | |
| GP400_22 | 650.00 mg | 75.00 mg | 3500.00 mg | 2500.00 mg | 3903.04 mg | 58.72 mg | Formulation fully dissolved, precipitation after cold storage |
| | 6.10% | 0.70% | 32.86% | 23.47% | 36.65% | 0.55% | |
| GP400_23 | 650.00 mg | 75.00 mg | 4000.00 mg | 2000.00 mg | 3903.04 mg | 58.72 mg | Formulation fully dissolved, precipitation after cold storage |
| | 6.10% | 0.70% | 37.56% | 18.78% | 36.65% | 0.55% | |
| GP400_24 | 650.00 mg | 75.00 mg | 4500.00 mg | 1500.00 mg | 3903.04 mg | 58.72 mg | Formulation fully dissolved, precipitation after cold storage |
| | 6.10% | 0.70% | 42.25% | 14.08% | 36.65% | 0.55% | |

**[Comparison of Formulations GP1450_1 to GP1450_24]**

| Formulation | Acetaminophen | Tramadol hydrochloride | Glycerin | PEG1450 | Water | Sucralose | Clear and transparent |
|---|---|---|---|---|---|---|---|
| GP1450_1 | 650.00 mg | 75.00 mg | 500.00 mg | 2500.00 mg | 6830.32 mg | 102.76 mg | Formulation fully dissolved, precipitation after cold storage |
| | 6.10% | 0.70% | 4.69% | 23.47% | 64.13% | 0.96% | |
| GP1450_2 | 650.00 mg | 75.00 mg | 1000.00 mg | 2000.00 mg | 6830.32 mg | 102.76 mg | Formulation fully dissolved, precipitation after cold storage |
| | 6.10% | 0.70% | 9.39% | 18.78% | 64.13% | 0.96% | |
| GP1450_3 | 650.00 mg | 75.00 mg | 1500.00 mg | 1500.00 mg | 6830.32 mg | 102.76 mg | Formulation fully dissolved, precipitation after cold storage |
| | 6.10% | 0.70% | 14.08% | 14.08% | 64.13% | 0.96% | |
| GP1450_4 | 650.00 mg | 75.00 mg | 500.00 mg | 3000.00 mg | 6342.44 mg | 95.42 mg | Formulation fully dissolved, precipitation after cold storage |
| | 6.10% | 0.70% | 4.69% | 28.17% | 59.55% | 0.90% | |
| GP1450_5 | 650.00 mg | 75.00 mg | 1000.00 mg | 2500.00 mg | 6342.44 mg | 95.42 mg | Formulation fully dissolved, precipitation after cold storage |
| | 6.10% | 0.70% | 9.39% | 23.47% | 59.55% | 0.90% | |
| GP1450_6 | 650.00 mg | 75.00 mg | 1500.00 mg | 2000.00 mg | 6342.44 mg | 95.42 mg | Formulation fully dissolved, precipitation after cold storage |
| | 6.10% | 0.70% | 14.08% | 18.78% | 59.55% | 0.90% | |
| GP1450_7 | 650.00 mg | 75.00 mg | 2000.00 mg | 1500.00 mg | 6342.44 mg | 95.42 mg | Formulation fully dissolved, precipitation after cold storage |
| | 6.10% | 0.70% | 18.78% | 14.08% | 59.55% | 0.90% | |
| GP1450_8 | 650.00 mg | 75.00 mg | 500.00 mg | 3500.00 mg | 5854.56 mg | 88.08 mg | Formulation fully dissolved, precipitation after cold storage |
| | 6.10% | 0.70% | 4.69% | 32.86% | 54.97% | 0.83% | |
| GP1450_9 | 650.00 mg | 75.00 mg | 1000.00 mg | 3000.00 mg | 5854.56 mg | 88.08 mg | Formulation fully dissolved, precipitation after cold storage |
| | 6.10% | 0.70% | 9.39% | 28.17% | 54.97% | 0.83% | |
| GP1450_10 | 650.00 mg | 75.00 mg | 2000.00 mg | 2000.00 mg | 5854.56 mg | 88.08 mg | Formulation fully dissolved, precipitation after cold storage |
| | 6.10% | 0.70% | 18.78% | 18.78% | 54.97% | 0.83% | |
| GP1450_11 | 650.00 mg | 75.00 mg | 2500.00 mg | 1500.00 mg | 5854.56 mg | 88.08 mg | Formulation fully dissolved, precipitation after cold storage |
| | 6.10% | 0.70% | 23.47% | 14.08% | 54.97% | 0.83% | |
| GP1450_12 | 650.00 mg | 75.00 mg | 1000.00 mg | 3500.00 mg | 5366.68 mg | 80.74 mg | No precipitation observed after cold storage |
| | 6.10% | 0.70% | 9.39% | 32.86% | 50.39% | 0.76% | |
| GP1450_13 | 650.00 mg | 75.00 mg | 1500.00 mg | 3000.00 mg | 5366.68 mg | 80.74 mg | No precipitation observed after cold storage |
| | 6.10% | 0.70% | 14.08% | 28.17% | 50.39% | 0.76% | |
| GP1450_14 | 650.00 mg | 75.00 mg | 1500.00 mg | 3500.00 mg | 4878.80 mg | 73.40 mg | No precipitation observed after cold storage |
| | 6.10% | 0.70% | 14.08% | 32.86% | 45.81% | 0.69% | |
| GP1450_15 | 650.00 mg | 75.00 mg | 2000.00 mg | 3000.00 mg | 4878.80 mg | 73.40 mg | No precipitation observed after cold storage |
| | 6.10% | 0.70% | 18.78% | 28.17% | 45.81% | 0.69% | |
| GP1450_16 | 650.00 mg | 75.00 mg | 2500.00 mg | 2500.00 mg | 4878.80 mg | 73.40 mg | Formulation fully dissolved, precipitation after cold storage |
| | 6.10% | 0.70% | 23.47% | 23.47% | 45.81 % | 0.69% | |
| GP1450_17 | 650.00 mg | 75.00 mg | 3000.00 mg | 2000.00 mg | 4878.80 mg | 73.40 mg | Formulation fully dissolved, precipitation after cold storage |
| | 6.10% | 0.70% | 28.17% | 18.78% | 45.81% | 0.69% | |
| GP1450_18 | 650.00 mg | 75.00 mg | 3500.00 mg | 1500.00 mg | 4878.80 mg | 73.40 mg | Formulation fully dissolved, precipitation after cold storage |
| | 6.10% | 0.70% | 32.86% | 14.08% | 45.81 % | 0.69% | |
| GP1450_19 | 650.00 mg | 75.00 mg | 2000.00 mg | 3500.00 mg | 4390.92 mg | 66.06 mg | No precipitation observed after cold storage |
| | 6.10% | 0.70% | 18.78% | 32.86% | 41.23% | 0.62% | |
| GP1450_20 | 650.00 mg | 75.00 mg | 2500.00 mg | 3000.00 mg | 4390.92 mg | 66.06 mg | No precipitation observed after cold storage |
| | 6.10% | 0.70% | 23.47% | 28.17% | 41.23% | 0.62% | |
| GP1450_21 | 650.00 mg | 75.00 mg | 3000.00 mg | 3000.00 mg | 3903.04 mg | 58.72 mg | No precipitation observed after cold storage |
| | 6.10% | 0.70% | 28.17% | 28.17% | 36.65% | 0.55% | |
| GP1450_22 | 650.00 mg | 75.00 mg | 3500.00 mg | 2500.00 mg | 3903.04 mg | 58.72 mg | Formulation fully dissolved, precipitation after cold storage |
| | 6.10% | 0.70% | 32.86% | 23.47% | 36.65% | 0.55% | |
| GP1450_23 | 650.00 mg | 75.00 mg | 4000.00 mg | 2000.00 mg | 3903.04 mg | 58.72 mg | Formulation fully dissolved, precipitation after cold storage |
| | 6.10% | 0.70% | 37.56% | 18.78% | 36.65% | 0.55% | |
| GP1450_24 | 650.00 mg | 75.00 mg | 4500.00 mg | 1500.00 mg | 3903.04 mg | 58.72 mg | Formulation fully dissolved, precipitation after cold storage |
| | 6.10% | 0.70% | 42.25% | 14.08% | 36.65% | 0.55% | |

**[Comparison of Formulations GP3350_1 to GP3350_24]**

| Formulation | Acetaminophen | Tramadol hydrochloride | Glycerin | PEG3350 | Water | Sucralose | Clear and transparent |
|---|---|---|---|---|---|---|---|
| GP3350_1 | 650.00 mg | 75.00 mg | 500.00 mg | 2500.00 mg | 6830.32 mg | 102.76 mg | Formulation fully dissolved, precipitation after cold storage |
| | 6.10% | 0.70% | 4.69% | 23.47% | 64.13% | 0.96% | |
| GP3350_2 | 650.00 mg | 75.00 mg | 1000.00 mg | 2000.00 mg | 6830.32 mg | 102.76 mg | Formulation fully dissolved, precipitation after cold storage |
| | 6.10% | 0.70% | 9.39% | 18.78% | 64.13% | 0.96% | |
| GP3350_3 | 650.00 mg | 75.00 mg | 1500.00 mg | 1500.00 mg | 6830.32 mg | 102.76 mg | Formulation fully dissolved, precipitation after cold storage |
| | 6.10% | 0.70% | 14.08% | 14.08% | 64.13 % | 0.96% | |
| GP3350_4 | 650.00 mg | 75.00 mg | 500.00 mg | 3000.00 mg | 6342.44 mg | 95.42 mg | Formulation fully dissolved, precipitation after cold storage |
| | 6.10% | 0.70% | 4.69% | 28.17% | 59.55% | 0.90% | |
| GP3350_5 | 650.00 mg | 75.00 mg | 1000.00 mg | 2500.00 mg | 6342.44 mg | 95.42 mg | Formulation fully dissolved, precipitation after cold storage |
| | 6.10% | 0.70% | 9.39% | 23.47% | 59.55% | 0.90% | |
| GP3350_6 | 650.00 mg | 75.00 mg | 1500.00 mg | 2000.00 mg | 6342.44 mg | 95.42 mg | Formulation fully dissolved, precipitation after cold storage |
| | 6.10% | 0.70% | 14.08% | 18.78% | 59.55% | 0.90% | |
| GP3350_7 | 650.00 mg | 75.00 mg | 2000.00 mg | 1500.00 mg | 6342.44 mg | 95.42 mg | Formulation fully dissolved, precipitation after cold storage |
| | 6.10% | 0.70% | 18.78% | 14.08% | 59.55% | 0.90% | |
| GP3350_8 | 650.00 mg | 75.00 mg | 500.00 mg | 3500.00 mg | 5854.56 mg | 88.08 mg | No precipitation observed after cold storage |
| | 6.10% | 0.70% | 4.69% | 32.86% | 54.97% | 0.83% | |
| GP3350_9 | 650.00 mg | 75.00 mg | 1000.00 mg | 3000.00 mg | 5854.56 mg | 88.08 mg | Formulation fully dissolved, precipitation after cold storage |
| | 6.10% | 0.70% | 9.39% | 28.17% | 54.97% | 0.83% | |
| GP3350_10 | 650.00 mg | 75.00 mg | 2000.00 mg | 2000.00 mg | 5854.56 mg | 88.08 mg | Formulation fully dissolved, precipitation after cold storage |
| | 6.10% | 0.70% | 18.78% | 18.78% | 54.97% | 0.83% | |
| GP3350_11 | 650.00 mg | 75.00 mg | 2500.00 mg | 1500.00 mg | 5854.56 mg | 88.08 mg | Formulation fully dissolved, precipitation after cold storage |
| | 6.10% | 0.70% | 23.47% | 14.08% | 54.97% | 0.83% | |
| GP3350_12 | 650.00 mg | 75.00 mg | 1000.00 mg | 3500.00 mg | 5366.68 mg | 80.74 mg | No precipitation observed after cold storage |
| | 6.10% | 0.70% | 9.39% | 32.86% | 50.39% | 0.76% | |
| GP3350_13 | 650.00 mg | 75.00 mg | 1500.00 mg | 3000.00 mg | 5366.68 mg | 80.74 mg | No precipitation observed after cold storage |
| | 6.10% | 0.70% | 14.08% | 28.17% | 50.39% | 0.76% | |
| GP3350_14 | 650.00 mg | 75.00 mg | 1500.00 mg | 3500.00 mg | 4878.80 mg | 73.40 mg | No precipitation observed after cold storage |
| | 6.10% | 0.70% | 14.08% | 32.86% | 45.81% | 0.69% | |
| GP3350_15 | 650.00 mg | 75.00 mg | 2000.00 mg | 3000.00 mg | 4878.80 mg | 73.40 mg | No precipitation observed after cold storage |
| | 6.10% | 0.70% | 18.78% | 28.17% | 45.81% | 0.69% | |
| GP3350_16 | 650.00 mg | 75.00 mg | 2500.00 mg | 2500.00 mg | 4878.80 mg | 73.40 mg | No precipitation observed after cold storage |
| | 6.10% | 0.70% | 23.47% | 23.47% | 45.81 % | 0.69% | |
| GP3350_17 | 650.00 mg | 75.00 mg | 3000.00 mg | 2000.00 mg | 4878.80 mg | 73.40 mg | Formulation fully dissolved, precipitation after cold storage |
| | 6.10% | 0.70% | 28.17% | 18.78% | 45.81% | 0.69% | |
| GP3350_18 | 650.00 mg | 75.00 mg | 3500.00 mg | 1500.00 mg | 4878.80 mg | 73.40 mg | Formulation fully dissolved, precipitation after cold storage |
| | 6.10% | 0.70% | 32.86% | 14.08% | 45.81 % | 0.69% | |
| GP3350_19 | 650.00 mg | 75.00 mg | 2000.00 mg | 3500.00 mg | 4390.92 mg | 66.06 mg | No precipitation observed after cold storage |
| | 6.10% | 0.70% | 18.78% | 32.86% | 41.23% | 0.62% | |
| GP3350_20 | 650.00 mg | 75.00 mg | 2500.00 mg | 3000.00 mg | 4390.92 mg | 66.06 mg | No precipitation observed after cold storage |
| | 6.10% | 0.70% | 23.47% | 28.17% | 41.23% | 0.62% | |
| GP3350_21 | 650.00 mg | 75.00 mg | 3000.00 mg | 3000.00 mg | 3903.04 mg | 58.72 mg | No precipitation observed after cold storage |
| | 6.10% | 0.70% | 28.17% | 28.17% | 36.65% | 0.55% | |
| GP3350_22 | 650.00 mg | 75.00 mg | 3500.00 mg | 2500.00 mg | 3903.04 mg | 58.72 mg | Formulation fully dissolved, precipitation after cold storage |
| | 6.10% | 0.70% | 32.86% | 23.47% | 36.65% | 0.55% | |
| GP3350_23 | 650.00 mg | 75.00 mg | 4000.00 mg | 2000.00 mg | 3903.04 mg | 58.72 mg | Formulation fully dissolved, precipitation after cold storage |
| | 6.10% | 0.70% | 37.56% | 18.78% | 36.65% | 0.55% | |
| GP3350_24 | 650.00 mg | 75.00 mg | 4500.00 mg | 1500.00 mg | 3903.04 mg | 58.72 mg | Formulation fully dissolved, precipitation after cold storage |
| | 6.10% | 0.70% | 42.25% | 14.08% | 36.65% | 0.55% | |

Solvent systems that the weight percentage of water is approximately 36.65 wt% to 54.97 wt%, that a single polyethylene glycol variant (PEG300, PEG400, PEG1450, or PEG3350) is employed, and that the weight percentage of glycerin is approximately 4.69 wt% to 28.17 wt%, form clear and transparent acetaminophen and tramadol compound oral solutions, whose ingredients are fully dissolved.

Specifically, no precipitation is observed, even after cold storage, in solutions based on formulations that the weight percentage of water is approximately 54.97 wt%, and that the weight percentage of PEG300 or PEG3350 is more than approximately 32 wt% to 33 wt%.

No precipitation is observed, even after cold storage, in solutions based on formulations that the weight percentage of water is approximately 50.39 wt%, and that the weight percentage of PEG400, PEG1450 or PEG3350 is more than approximately 28 wt% to 29 wt% or that the weight percentage of PEG300 is 32 wt% to 33 wt%.

No precipitation is observed, even after cold storage, in solutions based on formulations that the weight percentage of water is approximately 45.81 wt%, and that the weight percentage of PEG300, PEG400, or PEG1450 is more than approximately 28 wt% to 29 wt% or that the weight percentage of PEG3350 is 23 wt% to 24 wt%.

No precipitation is observed, even after cold storage, in solutions based on formulations that the weight percentage of water is approximately 41.23 wt%, and that the weight percentage of PEG400, PEG1450 or PEG3350 is more than approximately 28 wt% to 29 wt% or that the weight percentage of PEG300 is 32 wt% to 33 wt%.

No precipitation is observed, even after cold storage, in solutions based on formulations that the weight percentage of water is approximately 36.65 wt%, and that the weight percentage of polyethylene glycol is more than approximately 28 wt% to 29 wt%.

It is therefore concluded that a solvent system comprising water, glycerin, and a single polyethylene glycol variant, wherein the weight percentage of the single polyethylene glycol variant is higher than 28 wt%, forms an acetaminophen and tramadol compound oral solution whose ingredients are fully dissolved that no precipitation is observed.

### Example 13

The instant example relates to formulations employing a single polyethylene glycol variant for manufacturing acetaminophen and tramadol compound oral solutions, as well as determination on whether the solution is clear and transparent with the ingredients thereof fully dissolved.

In the instant example, Formulations GP300_25 to GP300_39, GP400_25 to GP400_39, GP1450_25 to GP1450_39, and GP3350_25 to GP3350_39 as listed in the table below (in an amount of 10 mL) are implemented for producing acetaminophen and tramadol compound oral solutions:

**[Comparison of Formulations GP300_25 to GP300_39]**

| Formulation | Acetaminophen | Tramadol hydrochloride | Glycerin | PEG300 | Water | Sucralose | Clear and transparent |
|---|---|---|---|---|---|---|---|
| GP300_25 | 650.00 mg | 75.00 mg | 0.00 mg | 3350.00 mg | 5854.56 mg | 88.08 mg | Formulation fully dissolved, precipitation after cold storage |
| | 6.50% | 0.75% | 0.00% | 33.50% | 58.55% | 0.88% | |
| GP300_26 | 650.00 mg | 75.00 mg | 350.00 mg | 3000.00 mg | 5854.56 mg | 88.08 mg | No precipitation observed after cold storage |
| | 6.50% | 0.75% | 3.50% | 30.00% | 58.55% | 0.88% | |
| GP300_27 | 650.00 mg | 75.00 mg | 350.00 mg | 3500.00 mg | 5366.68 mg | 80.74 mg | No precipitation observed after cold storage |
| | 6.50% | 0.75% | 3.50% | 35.00% | 53.67% | 0.81% | |
| GP300_28 | 650.00 mg | 75.00 mg | 850.00 mg | 3000.00 mg | 5366.68 mg | 80.74 mg | Formulation fully dissolved, precipitation after cold storage |
| | 6.50% | 0.75% | 8.50% | 30.00% | 53.67% | 0.81% | |
| GP300_29 | 650.00 mg | 75.00 mg | 850.00 mg | 3500.00 mg | 4878.80 mg | 73.40 mg | No precipitation observed after cold storage |
| | 6.50% | 0.75% | 8.50% | 35.00% | 48.79% | 0.73% | |
| GP300_30 | 650.00 mg | 75.00 mg | 1350.00 mg | 3000.00 mg | 4878.80 mg | 73.40 mg | No precipitation observed after cold storage |
| | 6.50% | 0.75% | 13.50% | 30.00% | 48.79% | 0.73% | |
| GP300_31 | 650.00 mg | 75.00 mg | 1500.00 mg | 2850.00 mg | 4878.80 mg | 73.40 mg | Formulation fully dissolved, precipitation after cold storage |
| | 6.50% | 0.75% | 15.00% | 28.50% | 48.79% | 0.73% | |
| GP300_32 | 650.00 mg | 75.00 mg | 1350.00 mg | 3500.00 mg | 4390.92 mg | 66.06 mg | No precipitation observed after cold storage |
| | 6.50% | 0.75% | 13.50% | 35.00% | 43.91% | 0.66% | |
| GP300_33 | 650.00 mg | 75.00 mg | 1500.00 mg | 3350.00 mg | 4390.92 mg | 66.06 mg | No precipitation observed after cold storage |
| | 6.50% | 0.75% | 15.00% | 33.50% | 43.91% | 0.66% | |
| GP300_34 | 650.00 mg | 75.00 mg | 1850.00 mg | 3000.00 mg | 4390.92 mg | 66.06 mg | No precipitation observed after cold storage |
| | 6.50% | 0.75% | 18.50% | 30.00% | 43.91% | 0.66% | |
| GP300_35 | 650.00 mg | 75.00 mg | 2000.00 mg | 2850.00 mg | 4390.92 mg | 66.06 mg | No precipitation observed after cold storage |
| | 6.50% | 0.75% | 20.00% | 28.50% | 43.91% | 0.66% | |
| GP300_36 | 650.00 mg | 75.00 mg | 2000.00 mg | 3350.00 mg | 3903.04 mg | 58.72 mg | No precipitation observed after cold storage |
| | 6.50% | 0.75% | 20.00% | 33.50% | 39.03% | 0.59% | |
| GP300_37 | 650.00 mg | 75.00 mg | 2350.00 mg | 3000.00 mg | 3903.04 mg | 58.72 mg | Formulation fully dissolved, trace precipitation after cold storage |
| | 6.50% | 0.75% | 23.50% | 30.00% | 39.03% | 0.59% | |
| GP300_38 | 650.00 mg | 75.00 mg | 2500.00 mg | 2850.00 mg | 3903.04 mg | 58.72 mg | Formulation fully dissolved, trace precipitation after cold storage |
| | 6.50% | 0.75% | 25.00% | 28.50% | 39.03% | 0.59% | |
| GP300_39 | 650.00 mg | 75.00 mg | 2850.00 mg | 2500.00 mg | 3903.04 mg | 58.72 mg | Formulation fully dissolved, precipitation after cold storage |
| | 6.50% | 0.75% | 28.50% | 25.00% | 39.03% | 0.59% | |

**[Comparison of Formulations GP400_25 to GP400_39]**

| Formulation | Acetaminophen | Tramadol hydrochloride | Glycerin | PEG400 | Water | Sucralose | Clear and transparent |
|---|---|---|---|---|---|---|---|
| GP400_25 | 650.00 mg | 75.00 mg | 0.00 mg | 3350.00 mg | 5854.56 mg | 88.08 mg | No precipitation observed after cold storage |
| | 6.50% | 0.75% | 0.00% | 33.50% | 58.55% | 0.88% | |
| GP400_26 | 650.00 mg | 75.00 mg | 350.00 mg | 3000.00 mg | 5854.56 mg | 88.08 mg | No precipitation observed after cold storage |
| | 6.50% | 0.75% | 3.50% | 30.00% | 58.55% | 0.88% | |
| GP400_27 | 650.00 mg | 75.00 mg | 350.00 mg | 3500.00 mg | 5366.68 mg | 80.74 mg | No precipitation observed after cold storage |
| | 6.50% | 0.75% | 3.50% | 35.00% | 53.67% | 0.81% | |
| GP400_28 | 650.00 mg | 75.00 mg | 850.00 mg | 3000.00 mg | 5366.68 mg | 80.74 mg | Formulation fully dissolved, precipitation after cold storage |
| | 6.50% | 0.75% | 8.50% | 30.00% | 53.67% | 0.81% | |
| GP400_29 | 650.00 mg | 75.00 mg | 850.00 mg | 3500.00 mg | 4878.80 mg | 73.40 mg | No precipitation observed after cold storage |
| | 6.50% | 0.75% | 8.50% | 35.00% | 48.79% | 0.73% | |
| GP400_30 | 650.00 mg | 75.00 mg | 1350.00 mg | 3000.00 mg | 4878.80 mg | 73.40 mg | Formulation fully dissolved, trace precipitation after cold storage |
| | 6.50% | 0.75% | 13.50% | 30.00% | 48.79% | 0.73% | |
| GP400_31 | 650.00 mg | 75.00 mg | 1500.00 mg | 2850.00 mg | 4878.80 mg | 73.40 mg | Formulation fully dissolved, precipitation after cold storage |
| | 6.50% | 0.75% | 15.00% | 28.50% | 48.79% | 0.73% | |
| GP400_32 | 650.00 mg | 75.00 mg | 1350.00 mg | 3500.00 mg | 4390.92 mg | 66.06 mg | No precipitation observed after cold storage |
| | 6.50% | 0.75% | 13.50% | 35.00% | 43.91% | 0.66% | |
| GP400_33 | 650.00 mg | 75.00 mg | 1500.00 mg | 3350.00 mg | 4390.92 mg | 66.06 mg | No precipitation observed after cold storage |
| | 6.50% | 0.75% | 15.00% | 33.50% | 43.91% | 0.66% | |
| GP400_34 | 650.00 mg | 75.00 mg | 1850.00 mg | 3000.00 mg | 4390.92 mg | 66.06 mg | Formulation fully dissolved, precipitation after cold storage |
| | 6.50% | 0.75% | 18.50% | 30.00% | 43.91% | 0.66% | |
| GP400_35 | 650.00 mg | 75.00 mg | 2000.00 mg | 2850.00 mg | 4390.92 mg | 66.06 mg | Formulation fully dissolved, precipitation after cold storage |
| | 6.50% | 0.75% | 20.00% | 28.50% | 43.91 % | 0.66% | |
| GP400_36 | 650.00 mg | 75.00 mg | 2000.00 mg | 3350.00 mg | 3903.04 mg | 58.72 mg | No precipitation observed after cold storage |
| | 6.50% | 0.75% | 20.00% | 33.50% | 39.03% | 0.59% | |
| GP400_37 | 650.00 mg | 75.00 mg | 2350.00 mg | 3000.00 mg | 3903.04 mg | 58.72 mg | No precipitation observed after cold storage |
| | 6.50% | 0.75% | 23.50% | 30.00% | 39.03% | 0.59% | |
| GP400_38 | 650.00 mg | 75.00 mg | 2500.00 mg | 2850.00 mg | 3903.04 mg | 58.72 mg | No precipitation observed after cold storage |
| | 6.50% | 0.75% | 25.00% | 28.50% | 39.03% | 0.59% | |
| GP400_39 | 650.00 mg | 75.00 mg | 2850.00 mg | 2500.00 mg | 3903.04 mg | 58.72 mg | Formulation fully dissolved, precipitation after cold storage |
| | 6.50% | 0.75% | 28.50% | 25.00% | 39.03% | 0.59% | |

**[Comparison of Formulations GP1450_25 to GP1450_39]**

| Formulation | Acetaminophen | Tramadol hydrochloride | Glycerin | PEG1450 | Water | Sucralose | Clear and transparent |
|---|---|---|---|---|---|---|---|
| GP1450_25 | 650.00 mg | 75.00 mg | 0.00 mg | 3350.00 mg | 5854.56 mg | 88.08 mg | No precipitation observed after cold storage |
| | 6.50% | 0.75% | 0.00% | 33.50% | 58.55% | 0.88% | |
| GP1450_26 | 650.00 mg | 75.00 mg | 350.00 mg | 3000.00 mg | 5854.56 mg | 88.08 mg | Formulation fully dissolved, precipitation after cold storage |
| | 6.50% | 0.75% | 3.50% | 30.00% | 58.55% | 0.88% | |
| GP1450_27 | 650.00 mg | 75.00 mg | 350.00 mg | 3500.00 mg | 5366.68 mg | 80.74 mg | No precipitation observed after cold storage |
| | 6.50% | 0.75% | 3.50% | 35.00% | 53.67% | 0.81% | |
| GP1450_28 | 650.00 mg | 75.00 mg | 850.00 mg | 3000.00 mg | 5366.68 mg | 80.74 mg | Formulation fully dissolved, precipitation after cold storage |
| | 6.50% | 0.75% | 8.50% | 30.00% | 53.67% | 0.81% | |
| GP1450_29 | 650.00 mg | 75.00 mg | 850.00 mg | 3500.00 mg | 4878.80 mg | 73.40 mg | No precipitation observed after cold storage |
| | 6.50% | 0.75% | 8.50% | 35.00% | 48.79% | 0.73% | |
| GP1450_30 | 650.00 mg | 75.00 mg | 1350.00 mg | 3000.00 mg | 4878.80 mg | 73.40 mg | No precipitation observed after cold storage |
| | 6.50% | 0.75% | 13.50% | 30.00% | 48.79% | 0.73% | |
| GP1450_31 | 650.00 mg | 75.00 mg | 1500.00 mg | 2850.00 mg | 4878.80 mg | 73.40 mg | Formulation fully dissolved, precipitation after cold storage |
| | 6.50% | 0.75% | 15.00% | 28.50% | 48.79% | 0.73% | |
| GP1450_32 | 650.00 mg | 75.00 mg | 1350.00 mg | 3500.00 mg | 4390.92 mg | 66.06 mg | No precipitation observed after cold storage |
| | 6.50% | 0.75% | 13.50% | 35.00% | 43.91% | 0.66% | |
| GP1450_33 | 650.00 mg | 75.00 mg | 1500.00 mg | 3350.00 mg | 4390.92 mg | 66.06 mg | No precipitation observed after cold storage |
| | 6.50% | 0.75% | 15.00% | 33.50% | 43.91% | 0.66% | |
| GP1450_34 | 650.00 mg | 75.00 mg | 1850.00 mg | 3000.00 mg | 4390.92 mg | 66.06 mg | No precipitation observed after cold storage |
| | 6.50% | 0.75% | 18.50% | 30.00% | 43.91% | 0.66% | |
| GP1450_35 | 650.00 mg | 75.00 mg | 2000.00 mg | 2850.00 mg | 4390.92 mg | 66.06 mg | No precipitation observed after cold storage |
| | 6.50% | 0.75% | 20.00% | 28.50% | 43.91% | 0.66% | |
| GP1450_36 | 650.00 mg | 75.00 mg | 2000.00 mg | 3350.00 mg | 3903.04 mg | 58.72 mg | No precipitation observed after cold storage |
| | 6.50% | 0.75% | 20.00% | 33.50% | 39.03% | 0.59% | |
| GP1450_37 | 650.00 mg | 75.00 mg | 2350.00 mg | 3000.00 mg | 3903.04 mg | 58.72 mg | No precipitation observed after cold storage |
| | 6.50% | 0.75% | 23.50% | 30.00% | 39.03% | 0.59% | |
| GP1450_38 | 650.00 mg | 75.00 mg | 2500.00 mg | 2850.00 mg | 3903.04 mg | 58.72 mg | No precipitation observed after cold storage |
| | 6.50% | 0.75% | 25.00% | 28.50% | 39.03% | 0.59% | |
| GP1450_39 | 650.00 mg | 75.00 mg | 2850.00 mg | 2500.00 mg | 3903.04 mg | 58.72 mg | Formulation fully dissolved, precipitation after cold storage |
| | 6.50% | 0.75% | 28.50% | 25.00% | 39.03% | 0.59% | |

**[Comparison of Formulations GP3350_25 to GP3350_39]**

| Formulation | Acetaminophen | Tramadol hydrochloride | Glycerin | PEG3350 | Water | Sucralose | Clear and transparent |
|---|---|---|---|---|---|---|---|
| GP3350_25 | 650.00 mg | 75.00 mg | 0.00 mg | 3350.00 mg | 5854.56 mg | 88.08 mg | No precipitation observed after cold storage |
| | 6.50% | 0.75% | 0.00% | 33.50% | 58.55% | 0.88% | |
| GP3350_26 | 650.00 mg | 75.00 mg | 350.00 mg | 3000.00 mg | 5854.56 mg | 88.08 mg | No precipitation observed after cold storage |
| | 6.50% | 0.75% | 3.50% | 30.00% | 58.55% | 0.88% | |
| GP3350_27 | 650.00 mg | 75.00 mg | 350.00 mg | 3500.00 mg | 5366.68 mg | 80.74 mg | No precipitation observed after cold storage |
| | 6.50% | 0.75% | 3.50% | 35.00% | 53.67% | 0.81% | |
| GP3350_28 | 650.00 mg | 75.00 mg | 850.00 mg | 3000.00 mg | 5366.68 mg | 80.74 mg | No precipitation observed after cold storage |
| | 6.50% | 0.75% | 8.50% | 30.00% | 53.67% | 0.81% | |
| GP3350_29 | 650.00 mg | 75.00 mg | 850.00 mg | 3500.00 mg | 4878.80 mg | 73.40 mg | No precipitation observed after cold storage |
| | 6.50% | 0.75% | 8.50% | 35.00% | 48.79% | 0.73% | |
| GP3350_30 | 650.00 mg | 75.00 mg | 1350.00 mg | 3000.00 mg | 4878.80 mg | 73.40 mg | No precipitation observed after cold storage |
| | 6.50% | 0.75% | 13.50% | 30.00% | 48.79% | 0.73% | |
| GP3350_31 | 650.00 mg | 75.00 mg | 1500.00 mg | 2850.00 mg | 4878.80 mg | 73.40 mg | Formulation fully dissolved, precipitation after cold storage |
| | 6.50% | 0.75% | 15.00% | 28.50% | 48.79% | 0.73% | |
| GP3350_32 | 650.00 mg | 75.00 mg | 1350.00 mg | 3500.00 mg | 4390.92 mg | 66.06 mg | No precipitation observed after cold storage |
| | 6.50% | 0.75% | 13.50% | 35.00% | 43.91% | 0.66% | |
| GP3350_33 | 650.00 mg | 75.00 mg | 1500.00 mg | 3350.00 mg | 4390.92 mg | 66.06 mg | No precipitation observed after cold storage |
| | 6.50% | 0.75% | 15.00% | 33.50% | 43.91% | 0.66% | |
| GP3350_34 | 650.00 mg | 75.00 mg | 1850.00 mg | 3000.00 mg | 4390.92 mg | 66.06 mg | Formulation fully dissolved, trace precipitation after cold storage |
| | 6.50% | 0.75% | 18.50% | 30.00% | 43.91% | 0.66% | |
| GP3350_35 | 650.00 mg | 75.00 mg | 2000.00 mg | 2850.00 mg | 4390.92 mg | 66.06 mg | Formulation fully dissolved, precipitation after cold storage |
| | 6.50% | 0.75% | 20.00% | 28.50% | 43.91 % | 0.66% | |
| GP3350_36 | 650.00 mg | 75.00 mg | 2000.00 mg | 3350.00 mg | 3903.04 mg | 58.72 mg | No precipitation observed after cold storage |
| | 6.50% | 0.75% | 20.00% | 33.50% | 39.03% | 0.59% | |
| GP3350_37 | 650.00 mg | 75.00 mg | 2350.00 mg | 3000.00 mg | 3903.04 mg | 58.72 mg | No precipitation observed after cold storage |
| | 6.50% | 0.75% | 23.50% | 30.00% | 39.03% | 0.59% | |
| GP3350_38 | 650.00 mg | 75.00 mg | 2500.00 mg | 2850.00 mg | 3903.04 mg | 58.72 mg | No precipitation observed after cold storage |
| | 6.50% | 0.75% | 25.00% | 28.50% | 39.03% | 0.59% | |
| GP3350_39 | 650.00 mg | 75.00 mg | 2850.00 mg | 2500.00 mg | 3903.04 mg | 58.72 mg | Formulation fully dissolved, precipitation after cold storage |
| | 6.50% | 0.75% | 28.50% | 25.00% | 39.03% | 0.59% | |

Solvent systems that the weight percentage of water is approximately 29.03 wt% to 58.55 wt%, that a single polyethylene glycol variant (PEG300, PEG400, PEG1450, or PEG3350) is employed, and that the weight percentage of the polyethylene glycol variant is approximately 28.5 wt% to 33.50 wt%, form clear and transparent acetaminophen and tramadol compound oral solutions, whose ingredients are fully dissolved.

Specifically, no precipitation is observed, even after cold storage, in solutions based on formulations that the weight percentage of water is approximately 58.55 wt%, and that the weight percentage of PEG400 or PEG3350 is more than approximately 30 wt% or that the weight percentage of PEG1450 is more than 35 wt%.

Specifically, no precipitation is observed, even after cold storage, in solutions based on formulations that the weight percentage of water is approximately 53.67 wt%, and that the weight percentage of PEG300, PEG400 or PEG1450 is more than 35 wt% or that the weight percentage of PEG3350 is more than 30 wt%.

No precipitation is observed, even after cold storage, in solutions based on formulations that the weight percentage of water is approximately 48.79 wt%, and that the weight percentage of PEG300, PEG1450 or PEG3350 is more than approximately 30 wt% or that the weight percentage of PEG400 is more than 35 wt%.

No precipitation is observed, even after cold storage, in solutions based on formulations that the weight percentage of water is approximately 43.91 wt%, and that the weight percentage of PEG300 or PEG1450 is more than approximately 28 wt% to 29 wt% or that the weight percentage of PEG400 or PEG3350 is more than approximately 33 wt% to 34 wt%.

No precipitation is observed, even after cold storage, in solutions based on formulations that the weight percentage of water is approximately 39.03 wt%, and that the weight percentage of PEG400, PEG1450 or PEG3350 is more than approximately 28 wt% to 29 wt% or that the weight percentage of PEG300 is more than approximately 33 wt% to 34 wt%.

It is therefore concluded that a solvent system comprising water, glycerin, and a single polyethylene glycol variant, wherein the weight percentage of the single polyethylene glycol variant is from 28.5 wt% to 33.50 wt%, forms an acetaminophen and tramadol compound oral solution whose ingredients are fully dissolved that no precipitation is observed.

### Example 14

The instant example relates to formulations employing multiple polyethylene glycol variants for manufacturing acetaminophen and tramadol compound oral solutions, as well as determination on whether the solution is clear and transparent with the ingredients thereof fully dissolved.

In the instant example, Formulations GMP_1 to GMP_45 and Formulations GMP_46 to GMP_49 as listed in the tables below (in an amount of 10 mL) are implemented for producing acetaminophen and tramadol compound oral solutions:

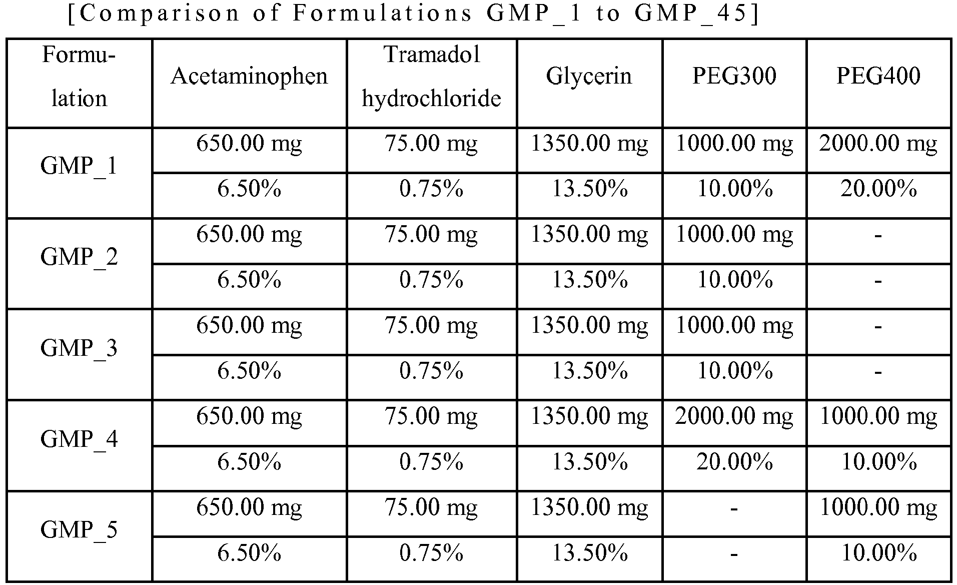

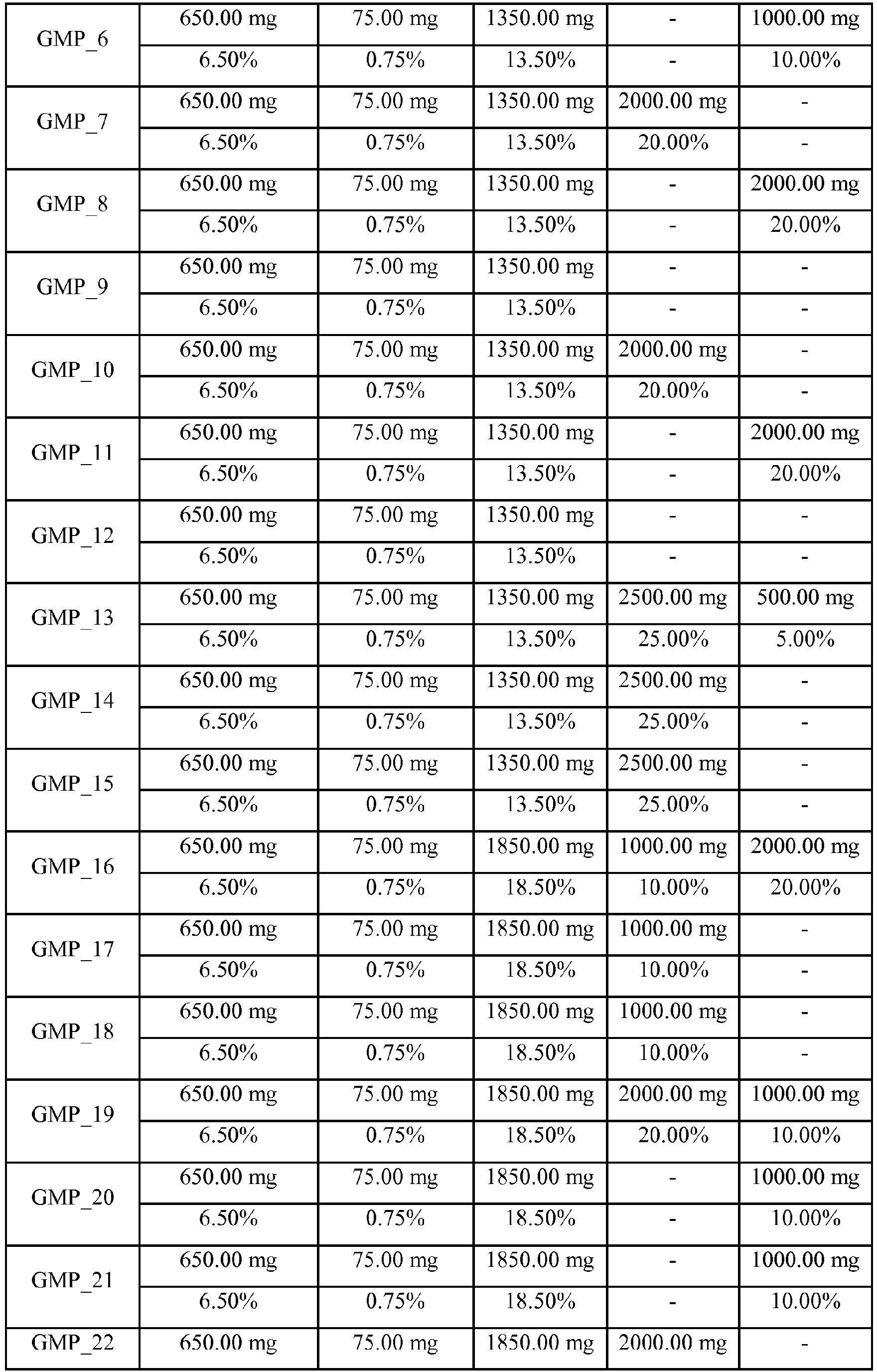

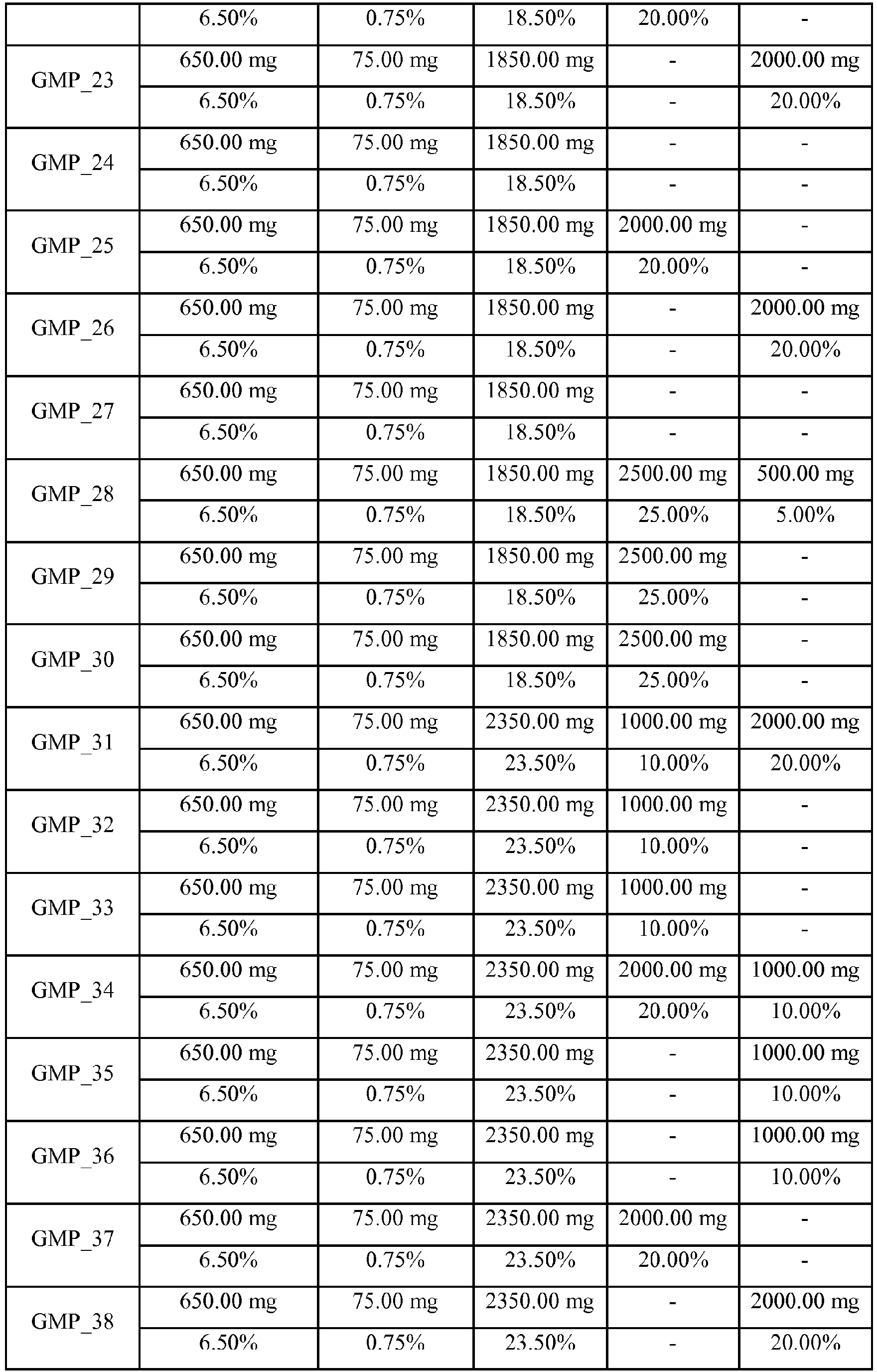

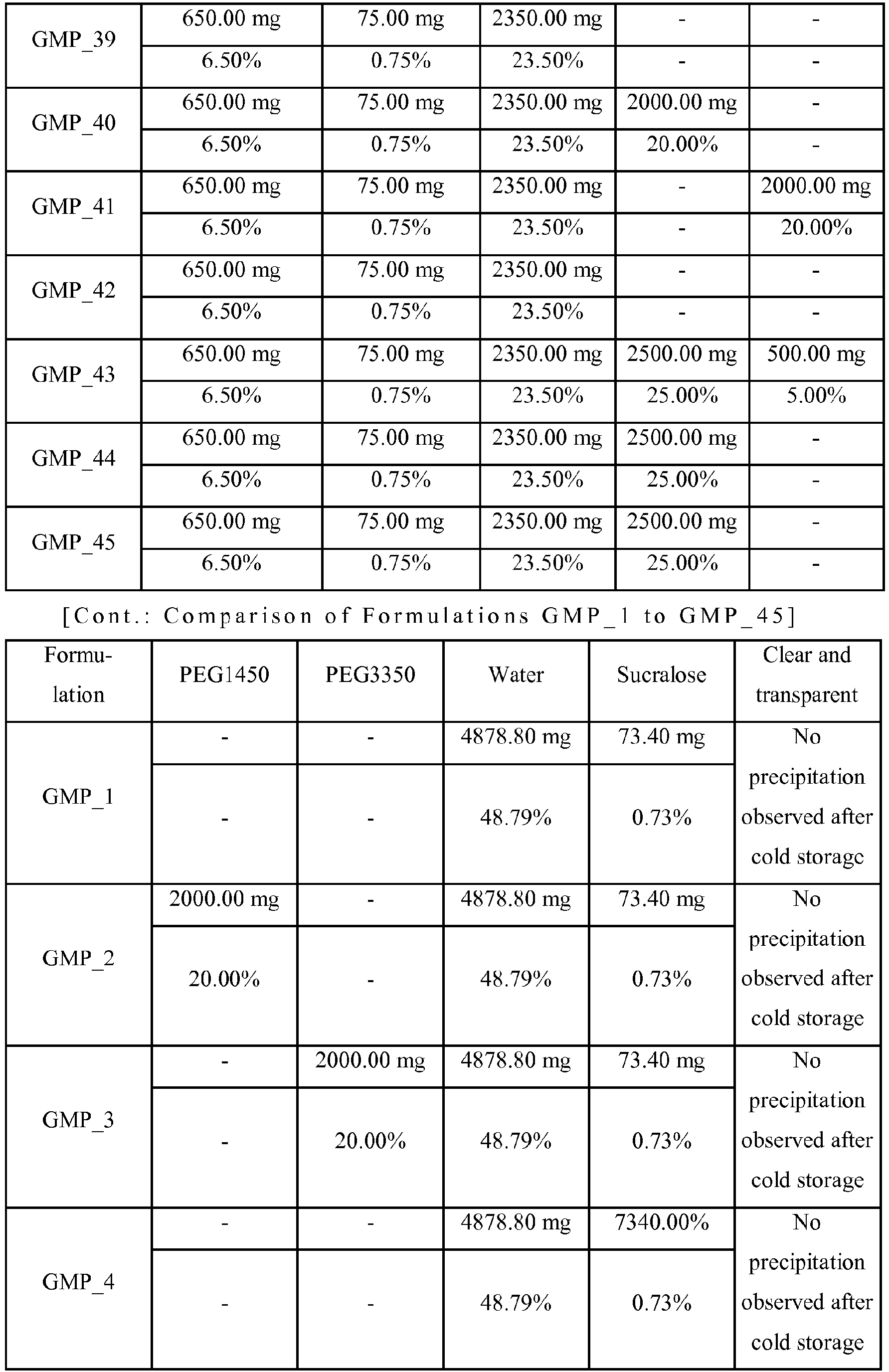

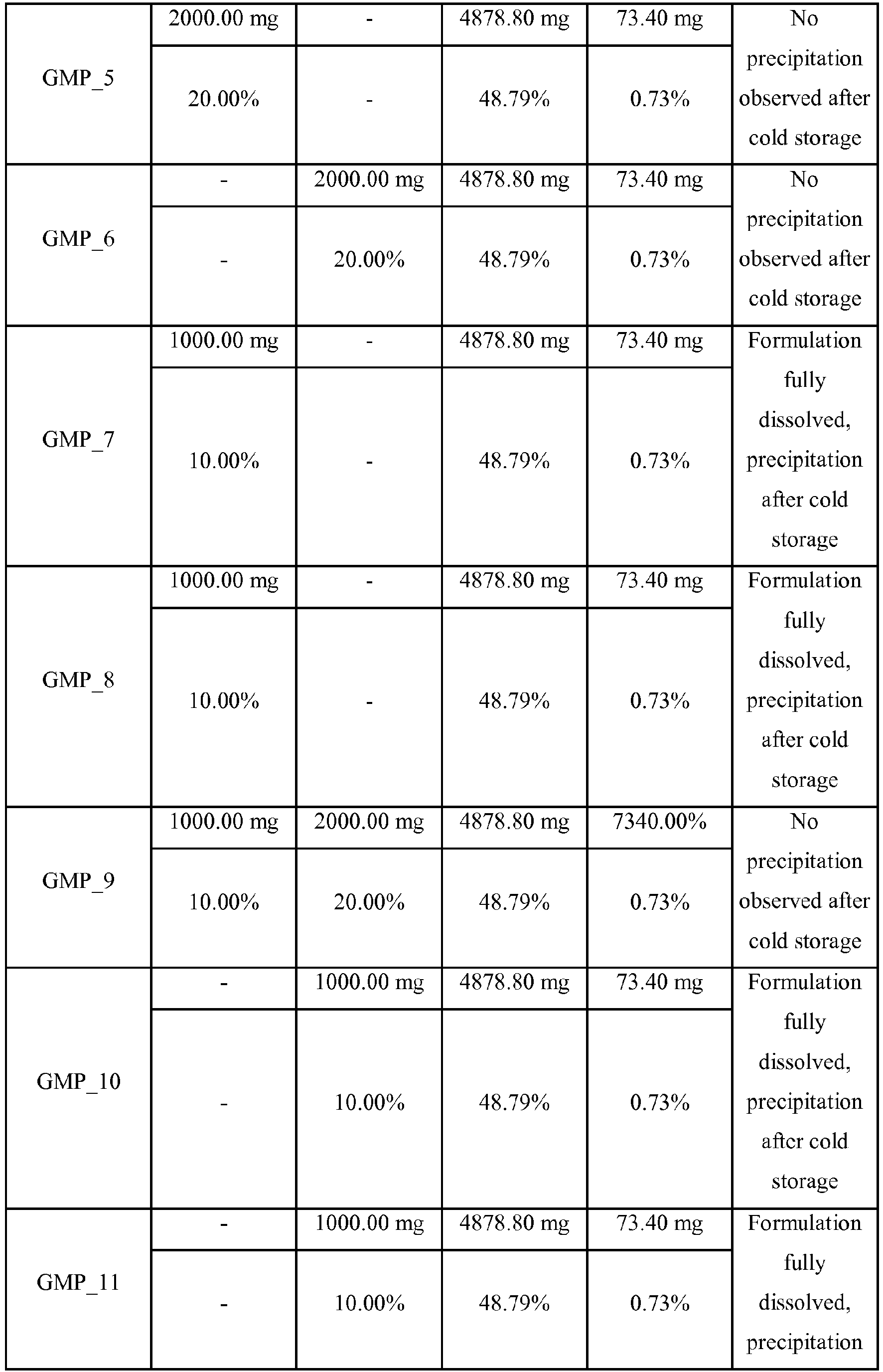

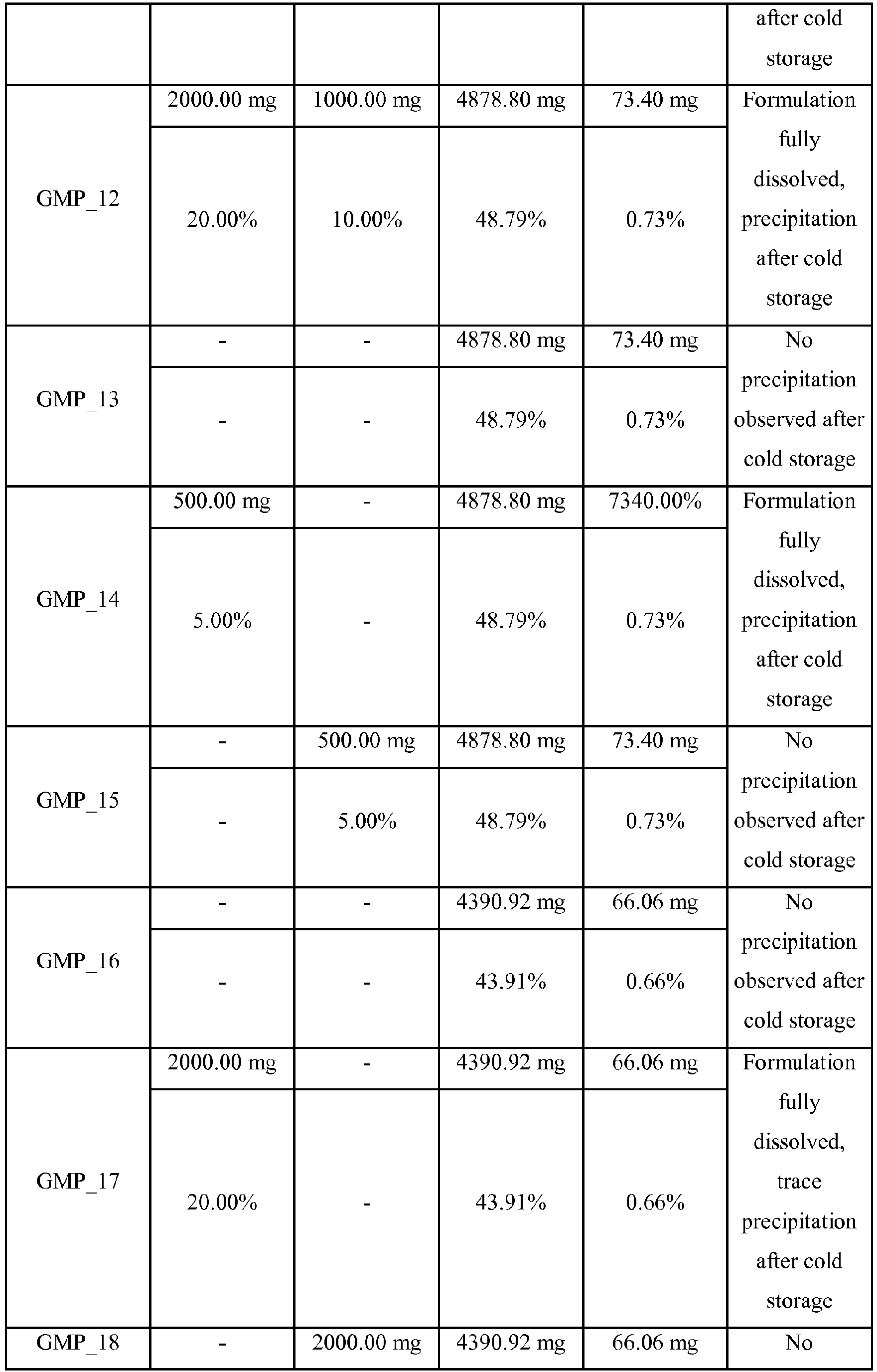

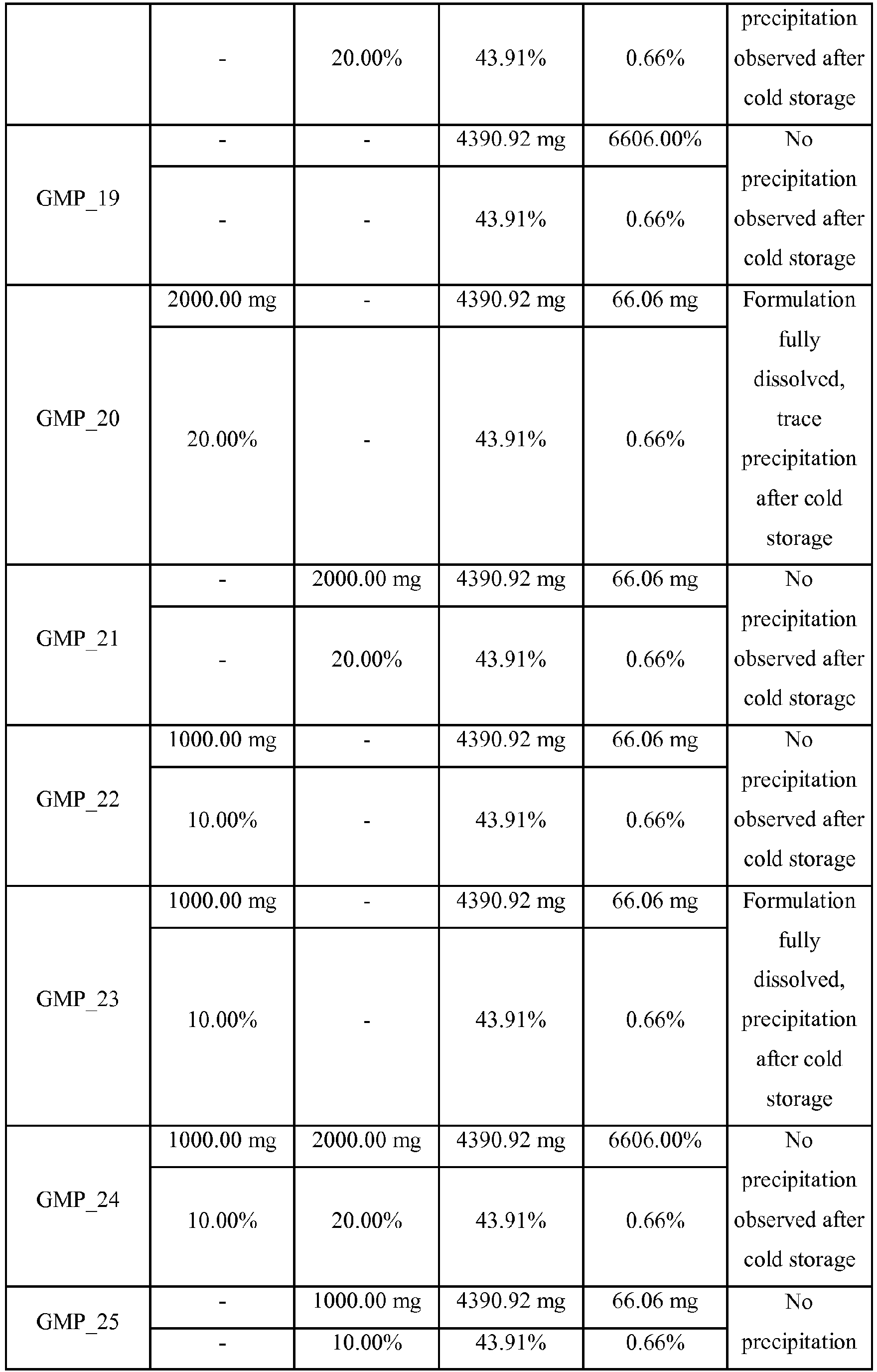

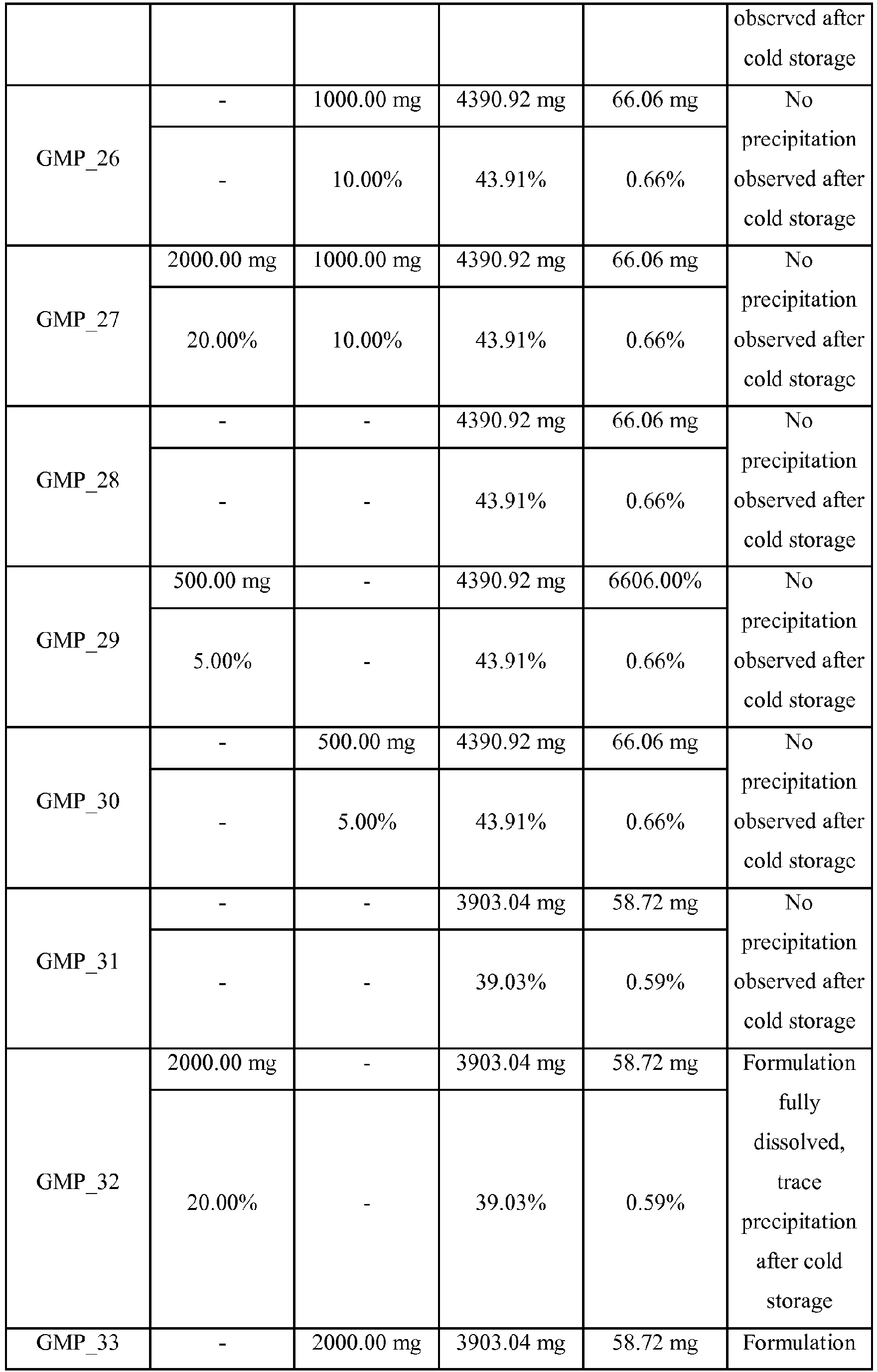

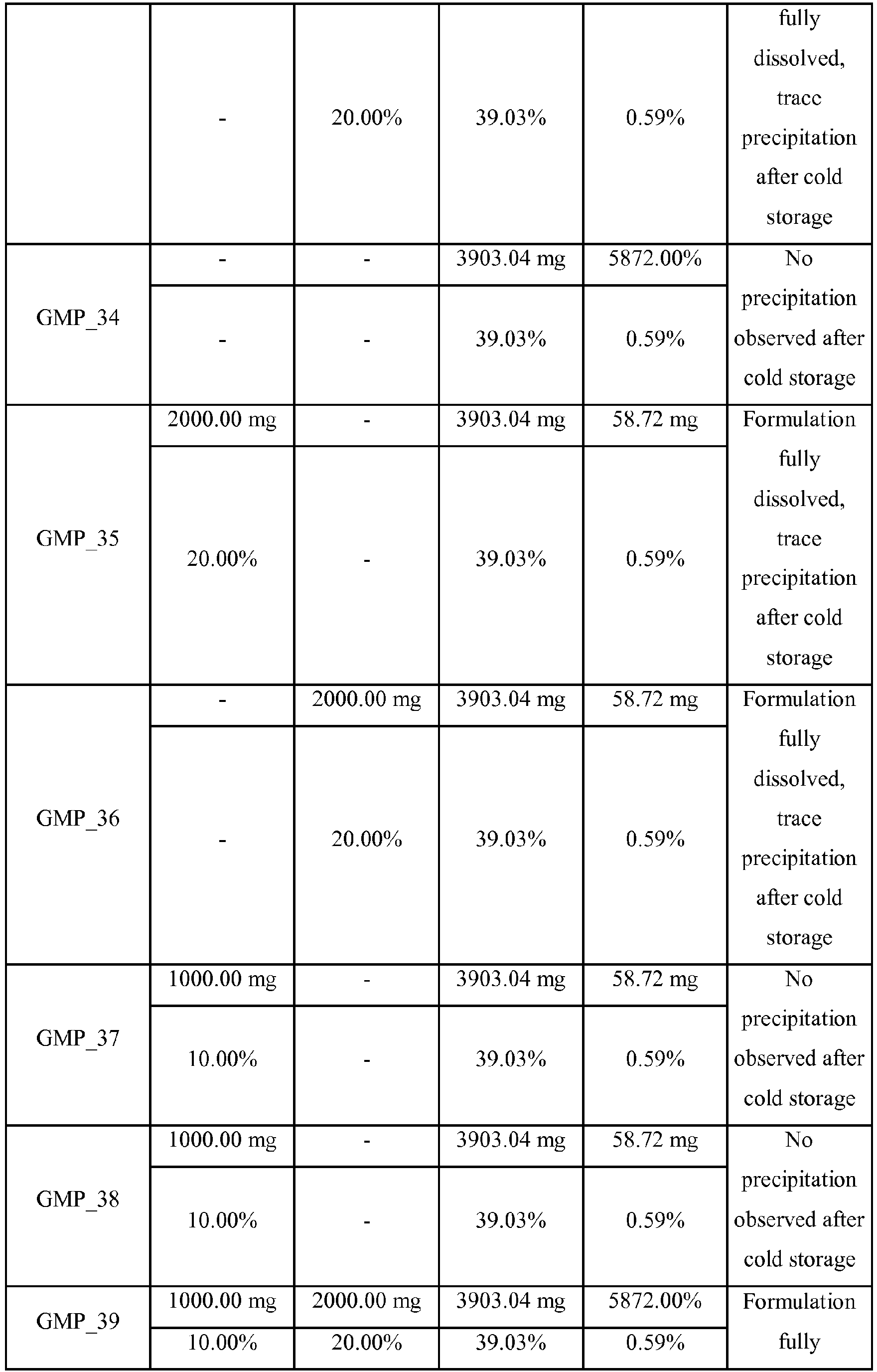

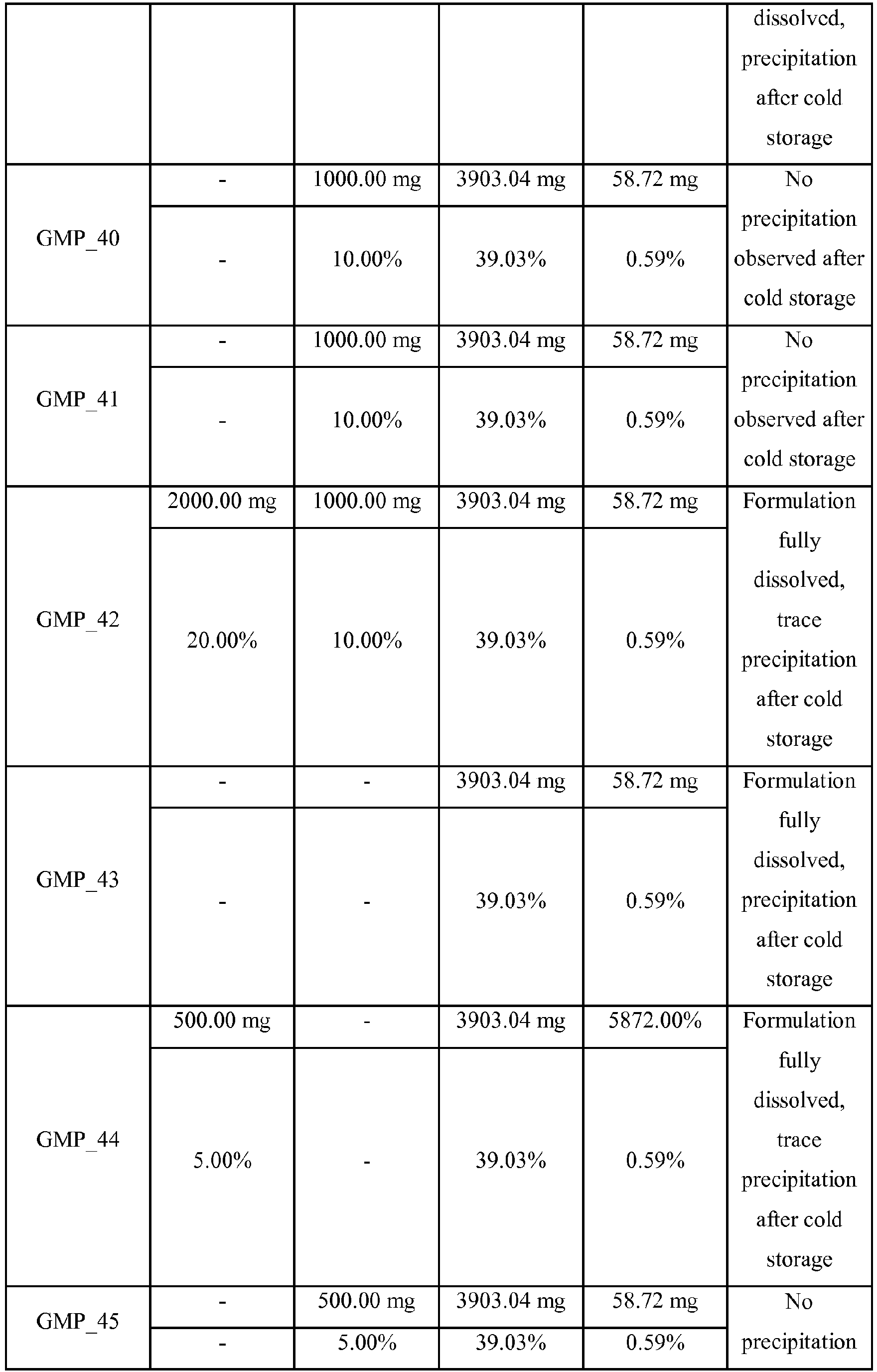

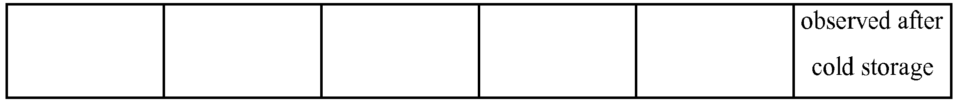

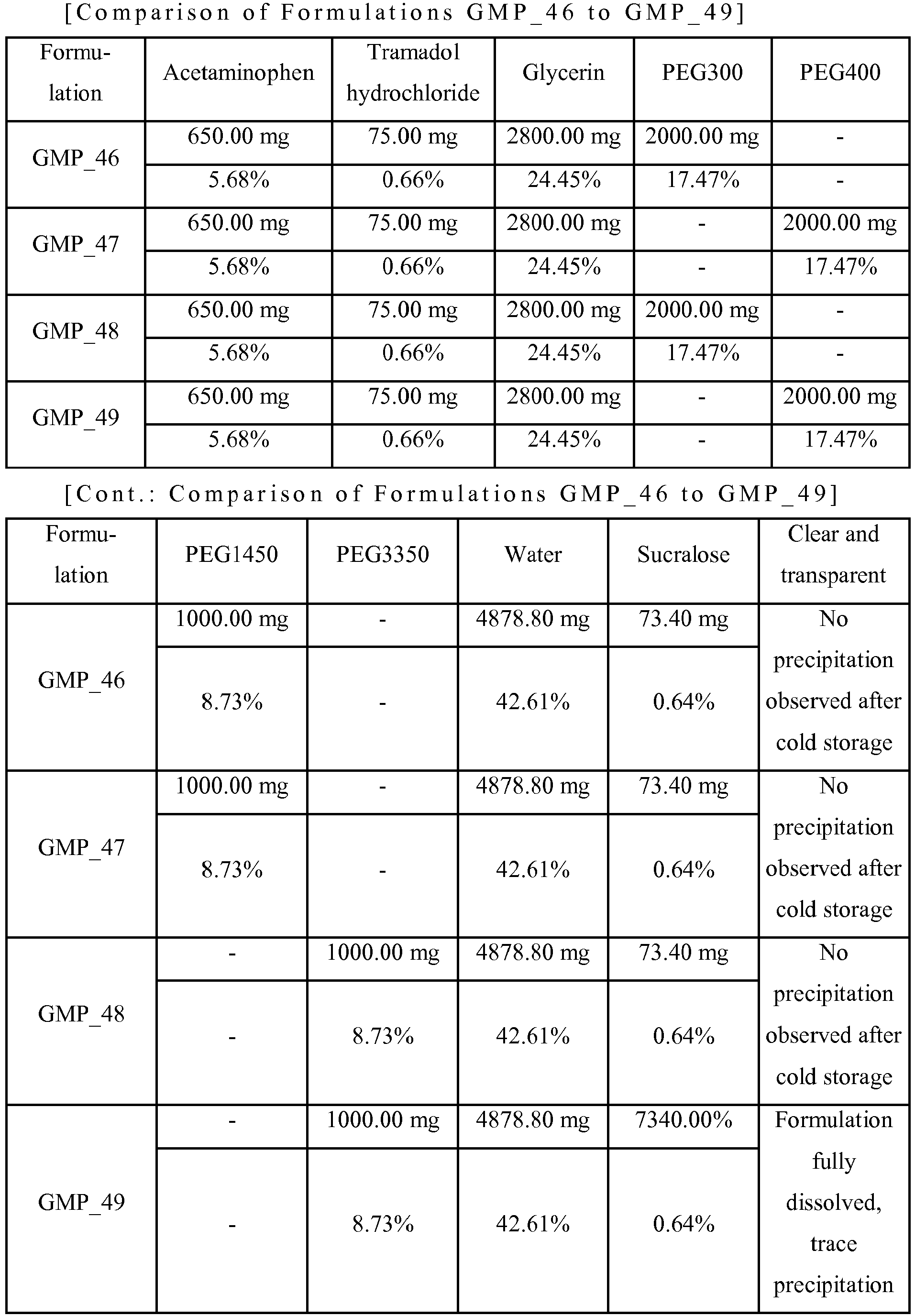

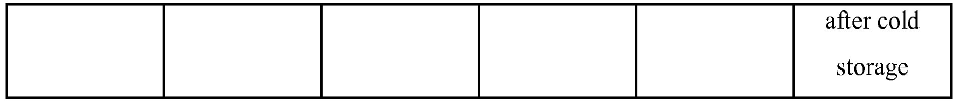

Solvent systems that the weight percentage of water is approximately 39.03 wt% to 48.79 wt%, that the weight percentage of glycerin is 13.50 wt% to 24.45 wt%, that multiple polyethylene glycol variants (selected from PEG300, PEG400, PEG1450, and PEG3350) are employed, and that the total weight percentage of the polyethylene glycol variants is approximately 30 wt%, form clear and transparent acetaminophen and tramadol compound oral solutions, whose ingredients are fully dissolved.

Specifically, no precipitation is observed, even after cold storage, in solutions based on formulations that the weight percentage of water is approximately 48.79 wt%, and that:
20 wt% of PEG1450 is employed in combination with PEG300 and PEG400;
20 wt% of PEG3350 is employed in combination with PEG300, PEG400 and PEG1450; or
25 wt% of PEG300 is employed in combination with and PEG3350.

It is therefore concluded that a solvent system comprising water, glycerin, and a single polyethylene glycol variant, wherein the weight percentage of the single polyethylene glycol variant is from 28.5 wt% to 33.50 wt%, forms an acetaminophen and tramadol compound oral solution whose ingredients are fully dissolved that no precipitation is observed.

Furthermore, no precipitation is observed, even after cold storage, in solutions based on formulations that the weight percentage of water is approximately 39.03 wt%, and that:
10 wt% of PEG300 is employed in combination with 20 wt% of PEG400, 20 wt% of PEG300 is employed in combination with 10 wt% of PEG1450, 20 wt% of PEG300 is employed in combination with 10 wt% of PEG1450, 20 wt% of PEG400 is employed in combination with 10 wt% of PEG1450, 20 wt% of PEG300 is employed in combination with 10 wt% of PEG3350, 20 wt% of PEG400 is employed in combination with 10 wt% of PEG3350, or 25 wt% of PEG300 is employed in combination with 5 wt% of PEG3350.

It is therefore concluded that a solvent system comprising water, glycerin, and multiple polyethylene glycol variants, wherein the weight percentage of water is approximately 39.03 wt% to 48.79 wt%, that the weight percentage of glycerin is 13.50 wt% to 24.45 wt%, and the total weight percentage of the multiple polyethylene glycol variants is approximately 30 wt%, forms an acetaminophen and tramadol compound oral solution whose ingredients are fully dissolved that no precipitation is observed.

### Example 15

The instant example relates to a formulation employing PEG400 and PEG1450 for manufacturing an acetaminophen and tramadol compound oral solution, as well as determination on whether the solution is clear and transparent with the ingredients thereof fully dissolved.

In the instant example, the integredients and ratio are as listed in the following table (in an amount of 5 mL) for producing the acetaminophen and tramadol compound oral solution:

| Formulation of Example 15 | Weight | Weight percentage |
|---|---|---|
| Acetaminophen | 325.00 mg | 5.82% |
| Tramadol hydrochloride | 37.50 mg | 0.67% |
| Glycerin | 1392.20 mg | 24.91% |
| PEG400 | 451.20 mg | 8.07% |
| PEG1450 | 1000.00 mg | 17.90% |
| Water | 2300.00 mg | 41.17% |
| Sucralose | 34.60 mg | 0.62% |
| Pineapple flavor | 23.00 mg | 0.41% |
| Yellow No. 4 | 1.16 mg | 0.02% |

When examined with the method as described in example 1, it is found that the solution prepared according to formulation of Example 15 is a clear and transparent solution.

The formulation of Example 15 provides a solvent system comprising water, glycerin, PEG400 and PEG1450 that forms an acetaminophen and tramadol compound oral solution whose ingredients are fully dissolved that no precipitation is observed.

As described with the foregoing examples, it evident that the present invention provides an acetaminophen and tramadol compound oral solution with specific solvent system. The acetaminophen and tramadol compound oral solution in accordance with the present invention is capable of providing the inventive effects of the present invention.

Furthermore, the present invention may further comprise a corrigent which may be a sweetener or a flavoring agent. Sucralose is used as a sweetener while pineapple flavor is used as a flavoring agent in foregoing examples. It is also feasible for the present invention to employ no sweetener, or, otherwise, to employ other sweeteners such as aspartame, acesulfame potassium, sodium cyclamate, sorbitol, or xylitol for the acetaminophen and tramadol compound oral solution. It is also feasible for the present invention to employ no flavoring agent, or, otherwise, to employ other flavoring agent such as orange flavor, lemon flavor, grape flavor, grapefruit flavor, mango flavor, vanilla flavor, blueberry flavor, or fruits flavor.

Additionally, the present invention may further comprise a coloring agent, such as Yellow No. 4, as used in the foregoing examples. It is also feasible for the present invention to employ no coloring agent, or, otherwise, to employ other pharmalogically acceptable coloring agent for the acetaminophen and tramadol compound oral solution.

## Claims

1. An acetaminophen and tramadol compound oral solution, **characterized in**, a total weight of the acetaminophen and tramadol compound oral solution being 100 wt%, comprising:
an acetaminophen having a weight percentage of 1.447 wt% to 6.702 wt%;
a tramadol having a weight percentage of 0.167 wt% to 0.773 wt%; and
a solvent system, weighted 90.828 wt% to 98.02 wt%, consisting of:
a glycerin, a water and at least one polyethylene glycol, wherein the polyethylene glycol has a weight percentage of 17 wt% to 65 wt% of the total weight of the acetaminophen and tramadol compound oral solution;
wherein the polyethylene glycol is selected from the group consisting of PEG300, PEG400, PEG1000, PEG1450 and PEG3350.

2. The acetaminophen and tramadol compound oral solution as claimed in claim 1, **characterized in that** the polyethylene glycol has a weight percentage of 17.06 wt% to 64.732 wt% of the total weight of the acetaminophen and tramadol compound oral solution.

3. The acetaminophen and tramadol compound oral solution as claimed in claim 1, **characterized in that**:
the polyethylene glycol is selected from the group consisting of:
PEG400 having a weight percentage of 17 wt% to 65 wt% of the total weight of the acetaminophen and tramadol compound oral solution;
PEG1000 having a weight percentage of 25 wt% to 26 wt% of the total weight of the acetaminophen and tramadol compound oral solution;
PEG1450 having a weight percentage of 19.55 wt% to 21.16 wt% of the total weight of the acetaminophen and tramadol compound oral solution; and
PEG3350 having a weight percentage of 17 wt% to 26 wt% of the total weight of the acetaminophen and tramadol compound oral solution.

4. The acetaminophen and tramadol compound oral solution as claimed in claim 2, **characterized in that**:
the polyethylene glycol has a weight percentage of 17.06 wt% to 34.12 wt% of the total weight of the acetaminophen and tramadol compound oral solution; and
the polyethylene glycol is selected from the group consisting of:
PEG400 having a weight percentage of 17.06 wt% to 34.12 wt% of the total weight of the acetaminophen and tramadol compound oral solution; and
PEG3350 having a weight percentage of 17.06 wt% to 25.59 wt% of the total weight of the acetaminophen and tramadol compound oral solution.

5. The acetaminophen and tramadol compound oral solution as claimed in claim 2, **characterized in that**:
the polyethylene glycol is PEG400; and
a ratio of a weight percentage of the glycerin to the weight percentage of the polyethylene glycol is less than 2.5.

6. The acetaminophen and tramadol compound oral solution as claimed in claim 5, **characterized in that** the water has a weight percentage of 3 wt% to 40 wt% of the total weight of the acetaminophen and tramadol compound oral solution.

7. The acetaminophen and tramadol compound oral solution as claimed in claim 5, **characterized in that** a ratio of a weight percentage of the glycerin to the weight percentage of the polyethylene glycol is from 0.3 to 2.4.

8. The acetaminophen and tramadol compound oral solution as claimed in claim 2, **characterized in that**:
the polyethylene glycol has a weight percentage of 25 wt% to 26 wt% of the total weight of the acetaminophen and tramadol compound oral solution;
the polyethylene glycol consists of PEG400 and PEG1450;
a ratio of a weight percentage of the PEG400 to a weight percentage of the PEG1450 is from 0.4 to 0.5; and
the glycerin has a weight percentage of 24.91 wt% to 28 wt% of the total weight of the acetaminophen and tramadol compound oral solution.

9. The acetaminophen and tramadol compound oral solution as claimed in claim 2, **characterized in that**:
the polyethylene glycol has a weight percentage of 28.5 wt% to 33.5 wt% of the total weight of the acetaminophen and tramadol compound oral solution; and
the water has a weight percentage of 39.03 wt% to 58.55 wt% of the total weight of the acetaminophen and tramadol compound oral solution.

10. The acetaminophen and tramadol compound oral solution as claimed in claim 2, **characterized in that**:
the polyethylene glycol has a weight percentage of 26.2 wt% to 30 wt% of the total weight of the acetaminophen and tramadol compound oral solution;
the glycerin has a weight percentage of 13.50 wt% to 24.45 wt% of the total weight of the acetaminophen and tramadol compound oral solution;
the water has a weight percentage of 39.03 wt% to 48.79 wt% of the total weight of the acetaminophen and tramadol compound oral solution; and
the polyethylene glycol is selected from the group consisting of:
PEG300 and PEG400;
PEG300 and PEG1450;
PEG300 and PEG3350;
PEG400 and PEG1450;
PEG400 and PEG3350; and
PEG1450 and PEG3350.

11. The acetaminophen and tramadol compound oral solution as claimed in any one of claims 1 to 10, **characterized in** further comprising:
a sweetener, a flavoring agent, a coloring agent or any combination thereof; wherein
the sweetener is selected from the group consisting of sucralose, aspartame, acesulfame potassium, sodium cyclamate, sorbitol and xylitol; and
the flavoring agent is selected from the group consisting of pineapple flavor, orange flavor, lemon flavor, grape flavor, grapefruit flavor, mango flavor, vanilla flavor, blueberry flavor and fruits flavor.

## Patentansprüche

1. Orale Lösung aus Acetaminophen und Tramadol Verbindung, **dadurch gekennzeichnet, dass** ein Gesamtgewicht der oralen Lösung der Acetaminophen und Tramadol Verbindung 100 Gewichts.-% ist, umfassend:
ein Acetaminophen aufweisend einen Gewichtsprozentsatz von 1,447 Gewichts.-% bis 6,702 Gewichts.-%;
ein Tramadol aufweisend einen Gewichtsprozentsatz von 0,167 Gewichts.-% bis 0,773 Gewichts.-%; und
ein Lösungsmittelsystem, gewichtet 90,828 Gewichts.-% bis 98,02 Gewichts.-%, bestehend aus:
einem Glycerin, einem Wasser und wenigstens einem Polyethylenglykol, wobei das Polyethylenglykol einen Gewichtsprozentsatz von 17 Gewichts.-% bis 65 Gewichts.-% des Gesamtgewichts von der oralen Lösung aus Acetaminophen und Tramadol Verbindung aufweist;
wobei das Polyethylenglykol ausgewählt ist, aus der Gruppe, bestehend aus PEG300, PEG400, PEG1000, PEG1450 und PEG3350.

2. Orale Lösung aus Acetaminophen und Tramadol Verbindung gemäß Anspruch 1, **dadurch gekennzeichnet, dass** Polyethylenglykol einen Gewichtsprozentsatz von 17,06 Gewichts.-% bis 64,732 Gewichts.-% von dem Gesamtgewicht von der oralen Lösung aus Acetaminophen und Tramadol Verbindung aufweist.

3. Orale Lösung aus Acetaminophen und Tramadol Verbindung gemäß Anspruch 1, **dadurch gekennzeichnet, dass**:
das Polyethylenglykol ausgewählt ist, aus der Gruppe, bestehend aus:
PEG400, aufweisend einen Prozentsatz von 17 Gewichts.-% bis 65 Gewichts.-% des Gesamtgewichts der oralen Lösung aus Acetaminophen und Tramadol Verbindung;
PEG1000, aufweisend einen Gewichtsprozentsatz von 25 Gewichts.-% bis 26 Gewichts.-% des Gesamtgewichts der oralen Lösung aus Acetaminophen und Tramadol Verbindung;
PEG1450, aufweisend einen Gewichtsprozentsatz von 19,55 Gewichts.-% bis 21,16 Gewichts.-% des Gesamtgewichts der oralen Lösung von Acetaminophen und Tramadol Verbindung; und
PEG3350, aufweisend einen Gewichtsprozentsatz von 17 Gewichts.-% bis 26 Gewichts.-% des Gesamtgewichts der oralen Lösung aus Acetaminophen und Tramadol Verbindung.

4. Orale Lösung aus Acetaminophen und Tramadol Verbindung gemäß Anspruch 2, **dadurch gekennzeichnet, dass**:
das Polyethylenglykol ein Gesamtprozentsatz von 17,06 Gewichts.-% bis 34,12 Gewichts.-% des Gesamtgewichts der oralen Lösung aus Acetaminophen und Tramadol Verbindung aufweist; und
das Polyethylenglykol ausgewählt ist, aus der Gruppe, bestehend aus:
PEG400, aufweisend einen Gewichtsprozentsatz von 17,06 Gewichts.-% bis 34,12 Gewichts.-% des Gesamtgewichts der oralen Lösung aus Acetaminophen und Tramadol Verbindung; und
PEG3350, aufweisend einen Gewichtsprozentsatz von 17,06 Gewichts.-% bis 25,59 Gewichts.-% des Gesamtgewichts der oralen Lösung aus Acetaminophen und Tramadol Verbindung.

5. Orale Lösung aus Acetaminophen und Tramadol Verbindung gemäß Anspruch 2, **dadurch gekennzeichnet, dass**:
das Polyethylenglykol PEG400 ist; und
ein Verhältnis eines Gewichtsprozentsatz von dem Glycerin zu dem Gewichtsprozentsatz von Polyethylenglykol weniger als 2.5 ist.

6. Orale Lösung aus Acetaminophen und Tramadol Verbindung gemäß Anspruch 5, **dadurch gekennzeichnet, dass**:
das Wasser einen Gewichtsprozentsatz von 3 Gewichts.-% bis 40 Gewichts.-% des Gesamtgewichts der oralen Lösung aus Acetaminophen und Tramadol Verbindung aufweist.

7. Orale Lösung aus Acetaminophen und Tramadol Verbindung gemäß Anspruch 5, **dadurch gekennzeichnet, dass**:
ein Verhältnis eines Gewichtsprozentsatz von Glycerin zu dem Gewichtsprozentsatz von Polyethylenglykol von 0,3 bis 2,4 ist.

8. Orale Lösung aus Acetaminophen und Tramadol Verbindung gemäß Anspruch 2, **dadurch gekennzeichnet, dass**:
das Polyethylenglykol einen Gewichtsprozentsatz von 25 Gewichts.-% bis 26 Gewichts.-% des Gesamtgewichts der oralen Lösung aus Acetaminophen und Tramadol Verbindung aufweist;
das Polyethylenglykol aus PEG400 und PEG1450 besteht;
ein Verhältnis eines Gewichtsprozentsatz der PEG400 zu einem Gewichtsprozentsatz von PEG1450 von 0,4 zu 0,5 ist; und
das Glycerin einen Gewichtsprozentsatz von 24,91 Gewichts.-% bis 28 Gewichts.-% des Gesamtgewichts der oralen Lösung aus Acetaminophen und Tramadol Verbindung aufweist.

9. Orale Lösung aus Acetaminophen und Tramadol Verbindung gemäß Anspruch 2, **dadurch gekennzeichnet, dass**:
das Polyethylenglykol einen Gewichtsprozentsatz von 28,5 Gewichts.-% bis 33,5 Gewichts.-% des Gesamtgewichts der oralen Lösung aus Acetaminophen und Tramadol Verbindung aufweist; und
das Wasser einen Gewichtsprozentsatz von 39,03 Gewichts.-% bis 58,55 Gewichts.-% des Gesamtgewichts der oralen Lösung der Acetaminophen und Tramadol Verbindung aufweist.

10. Orale Lösung aus Acetaminophen und Tramadol Verbindung gemäß Anspruch 2, **dadurch gekennzeichnet, dass**:
das Polyethylenglykol einen Gewichtsprozentsatz von 26,2 Gewichts.-% bis 30 Gewichts.-% des Gesamtgewichts der oralen Lösung aus Acetaminophen und Tramadol Verbindung aufweist;
das Glycerin einen Gewichtsprozentsatz von 13,50 Gewichts.-% bis 24,45 Gewichts.-% des Gesamtgewichts der oralen Lösung aus Acetaminophen und Tramadol Verbindung aufweist;
das Wasser einen Gewichtsprozentsatz von 39,03 Gewichts.-% bis 48,79 Gewichts.-% des Gesamtgewichts der oralen Lösung aus Acetaminophen und Tramadol Verbindung aufweist; und
das Polyethylenglykol ausgewählt ist, aus der Gruppe, bestehend aus:
PEG300 und PEG400;
PEG300 und PEG1450;
PEG300 und PEG3350;
PEG400 und PEG1450;
PEG400 und PEG3350; und
PEG1450 und PEG3350.

11. Orale Lösung aus Acetaminophen und Tramadol Verbindung gemäß irgendeinem der Ansprüche 1 bis 10, **dadurch gekennzeichnet, dass** sie ferner umfasst:
einen Süßstoff, einen Aromastoff, einen Farbstoff oder eine Kombination davon; wobei
der Süßstoff ausgewählt ist aus der Gruppe, bestehend aus Sucralose, Aspartam, Acesulfam-Kalium, Natriumcyclamat, Sorbit und Xylit; und
der Aromastoff ausgewählt ist aus der Gruppe, bestehend aus Ananasaroma, Orangenaroma, Zitronenaroma, Traubenaroma, Grapefruitaroma, Mangoaroma, Vanillearoma, Heidelbeeraroma und Fruchtaroma.

## Revendications

1. Solution orale de composé d'acétaminophène et de tramadol, **caractérisée en ce que** le poids total de la solution orale de composé d'acétaminophène et de tramadol de 100 % en poids comprenne :
de l'acétaminophène dont le pourcentage de poids est compris entre 1,447 % et 6,702 % en poids ;
du tramadol dont le pourcentage de poids est compris entre 0,167 % et 0,773 % en poids ; et
un système de solvant, pesant entre 90,828 % et 98,02 % en poids, consistant en :
de la glycérine, de l'eau et au moins un polyéthylène glycol, où le polyéthylène glycol représente entre 17 % et 65 % en poids du poids total de la solution orale de composé d'acétaminophène et de tramadol ;
où le polyéthylène glycol est sélectionné dans le groupe comprenant le PEG300, le PEG400, le PEG1000, le PEG1450 et le PEG3350.

2. Solution orale de composé d'acétaminophène et de tramadol selon la revendication 1, **caractérisée en ce que** le polyéthylène glycol représente entre 17,06 % et 64,732 % en poids du poids total de la solution orale de composé d'acétaminophène et de tramadol.

3. Solution orale de composé d'acétaminophène et de tramadol selon la revendication 1, **caractérisée en ce que** le polyéthylène glycol est sélectionné dans le groupe comprenant :
le PEG400 représentant entre 17 % et 65 % en poids du poids total de la solution orale de composé d'acétaminophène et de tramadol ;
le PEG1000 représentant entre 25 % et 26 % en poids du poids total de la solution orale de composé d'acétaminophène et de tramadol ;
le PEG1450 représentant entre 19,55 % et 21,16 % en poids du poids total de la solution orale de composé d'acétaminophène et de tramadol ; et
le PEG3350 représentant entre 17 % et 26 % en poids du poids total de la solution orale de composé d'acétaminophène et de tramadol.

4. Solution orale de composé d'acétaminophène et de tramadol selon la revendication 2, **caractérisée en ce que** :
le polyéthylène glycol représente entre 17,06 % et 34,12 % en poids du poids total de la solution orale de composé d'acétaminophène et de tramadol ; et
le polyéthylène glycol est sélectionné dans le groupe comprenant :
le PEG400 représentant entre 17,06 % et 34,12 % en poids du poids total de la solution orale de composé d'acétaminophène et de tramadol ; et
le PEG3350 représentant entre 17,06 % et 25,59 % en poids du poids total de la solution orale de composé d'acétaminophène et de tramadol.

5. Solution orale de composé d'acétaminophène et de tramadol selon la revendication 2, **caractérisée en ce que** :
le polyéthylène glycol est le PEG400 ; et
le ratio du pourcentage de poids de la glycérine par rapport au pourcentage de poids du polyéthylène glycol est inférieur à 2,5.

6. Solution orale de composé d'acétaminophène et de tramadol selon la revendication 5, **caractérisée en ce que** l'eau représente entre 3 % et 40 % en poids du poids total de la solution orale de composé d'acétaminophène et de tramadol.

7. Solution orale de composé d'acétaminophène et de tramadol selon la revendication 5, **caractérisée en ce que** le ratio du pourcentage de poids de la glycérine par rapport au pourcentage de poids du polyéthylène glycol est compris entre 0,3 et 2,4.

8. Solution orale de composé d'acétaminophène et de tramadol selon la revendication 2, **caractérisée en ce que** :
le polyéthylène glycol représente entre 25 % et 26 % en poids du poids total de la solution orale de composé d'acétaminophène et de tramadol ;
le polyéthylène glycol consiste en une association de PEG400 et PEG1450 ;
le ratio du pourcentage de poids du PEG400 par rapport au pourcentage de poids du PEG1450 est compris entre 0,4 et 0,5 ; et
la glycérine représente entre 24,91 % et 28 % en poids du poids total de la solution orale de composé d'acétaminophène et de tramadol.

9. Solution orale de composé d'acétaminophène et de tramadol selon la revendication 2, **caractérisée en ce que** :
le polyéthylène glycol représente entre 28,5 % et 33,5 % en poids du poids total de la solution orale de composé d'acétaminophène et de tramadol ; et
l'eau représente entre 39,03 % et 58,55 % en poids du poids total de la solution orale de composé d'acétaminophène et de tramadol.

10. Solution orale de composé d'acétaminophène et de tramadol selon la revendication 2, **caractérisée en ce que** :
le polyéthylène glycol représente entre 26,2 % et 30 % en poids du poids total de la solution orale de composé d'acétaminophène et de tramadol ;
la glycérine représente entre 13,50 % et 24,45 % en poids du poids total de la solution orale de composé d'acétaminophène et de tramadol ;
l'eau représente entre 39,03 % et 48,79 % en poids du poids total de la solution orale de composé d'acétaminophène et de tramadol ; et
le polyéthylène glycol est sélectionné dans le groupe comprenant :
le PEG300 et le PEG400 ;
le PEG300 et le PEG1450 ;
le PEG300 et le PEG3350 ;
le PEG400 et le PEG1450 ;
le PEG400 et le PEG3350 ; et
le PEG1450 et le PEG3350.

11. Solution orale de composé d'acétaminophène et de tramadol selon l'une quelconque des revendications 1 à 10, **caractérisée en ce qu'**elle contient en outre :
un édulcorant, un arôme, un colorant ou une combinaison de ces éléments ; où
l'édulcorant est sélectionné dans le groupe comprenant le sucralose, l'aspartame, l'acésulfame potassium, le cyclamate de sodium, le sorbitol et le xylitol ; et
l'arôme est sélectionné dans le groupe comprenant l'arôme d'ananas, l'arôme d'orange, l'arôme de citron, l'arôme de raisin, l'arôme de pamplemousse, l'arôme de mangue, l'arôme de vanille, l'arôme de myrtille et l'arôme de fruits.
